# EUROPEAN PATENT APPLICATION

(11) **EP 4 210 064 A1**
(43) Date of publication of application: **12.07.2023**
(21) Application number: 21864388.0
(22) Date of filing: 02.09.2021
(51) Int. Cl.: G16H 10/60, G16H 20/00, G16H 50/30, G06N 20/00, A61B 5/02, A61B 5/022, A61B 5/0245, A61B 5/08, A61B 5/145, A61B 5/1455

(54) **BIOMETRIC INFORMATION COMPUTING SYSTEM, SERVER, AND DATA STRUCTURE**

(30) Priority: 03.09.2020 JP 2020148047; 03.09.2020 JP 2020148048; 02.03.2021 JP 2021032880
(71) Applicant: SSST Co., LTD., Ueda-shi, Nagano 386-0017 (JP)
(72) Inventor: KURASAWA, Shintaro, Ueda-shi, Nagano 386-0017 (JP); CHINO, Shun, Ueda-shi, Nagano 386-0017 (JP)
(74) Representative: Plasseraud IP
(86) International application number: PCT/JP2021/032231
(87) International publication number: WO 2022/050333

(57) **Abstract**

[Problem]

Provided is a biometric information computing system that allows attempting improvement of accuracy in evaluating biometric information.

[Solution]

A biometric information computing system for evaluating biometric information of a user includes obtaining means that obtains first evaluation data and second evaluation data based on a pulse wave of the user, a database, and generating means that generates a first evaluation result including first biometric information for the first evaluation data by referring to the database. The database stores classification information generated using a plurality of training data, and the training data is a pair of input data based on a preliminarily obtained training pulse wave and reference data including biometric information associated with the input data.

## Description

### TECHNICAL FIELD

The present invention relates to a biometric information computing system, a server, and a data structure.

### BACKGROUND ART

Conventionally, as a method for evaluating biometric information such as a blood glucose level of a user, for example, a method as disclosed in Patent Document 1 has been proposed.

Patent Document 1 discloses a biometric information estimation device and its method in which an acceleration pulse wave of a user is measured, and blood glucose level information of the user is extracted with a non-invasive method from waveform information on the measured acceleration pulse wave based on a correlation between a blood glucose level measured by an invasive measurement method and a simultaneously-measured acceleration pulse wave without using spectroscopic analysis.

Patent Document 1: Japanese Patent No. 6544751

### DISCLOSURE OF THE INVENTION

### PROBLEMS TO BE SOLVED BY THE INVENTION

Here, the improvement of the accuracy in the evaluation of the biometric information of the user has been desired.

Therefore, the present invention is devised in consideration of the above-described problem, and has an object to provide a biometric information computing system, a server, and a data structure capable of attempting improvement of accuracy in an evaluation of biometric information.

### SOLUTIONS TO THE PROBLEMS

A biometric information computing system according to the first invention is a biometric information computing system for evaluating biometric information of a user, and includes: obtaining means that obtains first evaluation data and second evaluation data based on a pulse wave of the user; a database that stores classification information generated using a plurality of training data, the training data being a pair of input data based on a preliminarily obtained training pulse wave and reference data including biometric information associated with the input data; and generating means that generates a first evaluation result including first biometric information for the first evaluation data by referring to the database.

In the biometric information computing system according to the second invention, in the first invention, the generating means includes generating a second evaluation result including second biometric information for the second evaluation data, and the second biometric information is different in kind from the first biometric information.

The biometric information computing system according to the third invention, in the second invention, further includes storage means that stores the first evaluation result and the second evaluation result.

In the biometric information computing system according to the fourth invention, in the second invention, the obtaining means obtains the first evaluation data and the second evaluation data by performing mutually different kinds of processes on one pulse wave data corresponding to any of a velocity pulse wave and an acceleration pulse wave based on the pulse wave of the user.

In the biometric information computing system according to the fifth invention, in the second invention, the classification information includes first classification information and second classification information generated using different kinds of the training data, and the generating means includes: generating the first evaluation result for the first evaluation data by referring to the first classification information; and generating the second evaluation result for the second evaluation data by referring to the second classification information.

The biometric information computing system according to the sixth invention, in the second invention, includes comprehensive evaluation means that obtains additional information indicating a feature of the user and generates a comprehensive evaluation result comprehensively evaluating the feature of the user based on the first evaluation result, the second evaluation result, and the additional information.

In the biometric information computing system according to the seventh invention, in the first invention, the classification information includes a plurality of pieces of attribute-based classification information calculated using the mutually different training data, and the generating means includes: selecting means that selects first classification information among the plurality of pieces of attribute-based classification information by referring to the second evaluation data; and attribute-based generating means that generates the first evaluation result for the first evaluation data by referring to the first classification information.

In the biometric information computing system according to the eighth invention, in the seventh invention, the obtaining means includes: obtaining data corresponding to a velocity pulse wave based on the pulse wave as the first evaluation data; and obtaining data corresponding to an acceleration pulse wave based on the pulse wave as the second evaluation data.

In the biometric information computing system according to the ninth invention, in the first invention, the generating means includes: selecting first classification information among the classification information based on a feature of the pulse wave; and generating the first evaluation result for the first evaluation data by referring to the first classification information.

The biometric information computing system according to the tenth invention, in the third invention, includes calculating means that generates a comprehensive evaluation result comprehensively evaluating a feature of the user based on the first evaluation result and the second evaluation result stored by the storage means.

In the biometric information computing system according to the eleventh invention, in the third invention, the storage means includes: obtaining a determination result determined by the user for contents of the first evaluation result and the second evaluation result; and mutually associating and storing the determination result, the first evaluation result, and the second evaluation result.

The biometric information computing system according to the twelfth invention, in the eleventh invention, includes updating means that updates the classification information based on the determination result, the first evaluation result, and the second evaluation result stored by the storage means.

The biometric information computing system according to the thirteenth invention, in the first invention, includes a server that stores the database and generates the first evaluation result by the generating means; and a biometric information computing device that receives the first evaluation result from the server and displays the first evaluation result.

A server according to the fourteenth invention stores the first evaluation result according to the first invention.

A data structure according to the fifteenth invention is a data structure used by a computer that includes a display unit, a control unit, and a storing unit, the data structure is stored in the storing unit, and the data structure includes the first evaluation result and the second evaluation result generated by the biometric information computing system according to the second invention. The first evaluation result and the second evaluation result are used when the control unit generates a comprehensive evaluation result comprehensively evaluating a feature of the user.

### EFFECTS OF THE INVENTION

According to the first invention to the thirteenth invention, the obtaining means obtains the first evaluation data and the second evaluation data based on the pulse wave of the user. The generating means generates the first evaluation result including the first biometric information for the first evaluation data. That is, a plurality of evaluation data are obtained from one pulse wave of the user. Therefore, in obtaining the respective evaluation data, the variation caused by the measurement condition of the pulse wave can be eliminated. Accordingly, the improvement of the accuracy in the evaluation of the biometric information can be attempted.

According to the first invention to the thirteenth invention, the generating means refers to the database, and generates the first evaluation result. The database stores the classification information generated using a plurality of training data. Therefore, in generating the evaluation result, the quantitative evaluation result can be generated based on the association between the feature of the pulse wave proven in the past and the biometric information. Accordingly, the variation of the evaluation due to the subjective view of the user or the like can be suppressed.

Especially, according to the thirteenth invention, the server generates the first evaluation result by the generating means. The biometric information computing device receives the first evaluation result from the server, and displays the first evaluation result. Therefore, for the biometric information computing device, the load in generating the evaluation result can be reduced. Accordingly, the convenience of the biometric information computing device can be improved. The need for storing the database in the biometric information computing device can be eliminated. Accordingly, the data storage capacity of the biometric information computing device can be substantially reduced. Additionally, by storing the database in the server, the evaluation result generated with reference to one piece of the classification information can be output to a plurality of biometric information computing devices. Accordingly, the huge amount of time and money for updating the database for each biometric information computing device in association with the maintenance such as an update of the database can be reduced.

According to the fourteenth invention, the server can store the first evaluation result after attempting the improvement of accuracy in the evaluation of the biometric information.

According to the fifteenth invention, the data structure includes the first evaluation result and the second evaluation result after attempting the improvement of accuracy in the evaluation of the biometric information, and can be used for generating the comprehensive evaluation result.

### BRIEF DESCRIPTION OF THE DRAWINGS

Fig. 1 is a schematic diagram illustrating an example of a biometric information computing system in a first embodiment.
Fig. 2 is a schematic diagram illustrating an example of an operation of the biometric information computing system in the first embodiment.
Fig. 3A is a schematic diagram illustrating an example of classification information, and Fig. 3B is a schematic diagram illustrating another example of the classification information.
Fig. 4A to Fig. 4D are schematic diagrams illustrating examples of processing to sensor data.
Fig. 5A is a schematic diagram illustrating an example of a configuration of a biometric information computing device, and Fig. 5B is a schematic diagram illustrating an example of functions of the biometric information computing device.
Fig. 6A and Fig. 6B are schematic diagrams illustrating an example of a sensor.
Fig. 7 is a flowchart illustrating an example of the operation of the biometric information computing system in the first embodiment.
Fig. 8 is a schematic diagram illustrating an example of an operation of a biometric information computing system in a second embodiment.
Fig. 9 is a schematic diagram illustrating a modification of the operation of the biometric information computing system in the second embodiment.
Fig. 10 is a schematic diagram illustrating an example of an operation of a biometric information computing system in a third embodiment.
Fig. 11 is a schematic diagram illustrating an example of an operation of a biometric information computing system in a fourth embodiment.
Fig. 12 is a schematic diagram illustrating a classification example of data corresponding to an acceleration pulse wave.
Fig. 13 is a schematic diagram illustrating a classification example of data corresponding to a velocity pulse wave.
Fig. 14 is a schematic diagram illustrating a first modification of the operation of the biometric information computing system in the fourth embodiment.
Fig. 15 is a schematic diagram illustrating a second modification of the operation of the biometric information computing system in the fourth embodiment.
Fig. 16 is a schematic diagram illustrating a third modification of the operation of the biometric information computing system in the fourth embodiment.
Fig. 17 is a schematic diagram illustrating an example of an operation of a biometric information computing system in a fifth embodiment.
Fig. 18 is a schematic diagram illustrating an example of an operation of a biometric information computing system in a sixth embodiment.
Fig. 19 is a drawing illustrating a sequence for achieving a biometric information computing program of an electronic device in a seventh embodiment.
Fig. 20 is a schematic diagram illustrating an example of a first extracting unit and a first data obtaining unit.
Fig. 21 is a schematic diagram illustrating an example of a second extracting unit and a second data obtaining unit.
Fig. 22 is a flowchart illustrating an example of an operation of a biometric information computing system in the seventh embodiment.
Fig. 23 is a drawing illustrating a sequence for achieving a biometric information computing program of an electronic device in an eighth embodiment.
Fig. 24 is a schematic diagram illustrating an example of a classification data extracting unit.
Fig. 25 is a schematic diagram illustrating an example of an evaluation-aimed data extracting unit.
Fig. 26 is a flowchart illustrating an example of an operation of a biometric information computing system in the eighth embodiment.

### DESCRIPTION OF PREFERRED EMBODIMENTS

The following describes examples of a biometric information computing system, a server, and a data structure in the embodiments of the present invention by referring to the drawings.

### (First Embodiment: Biometric Information Computing System 100)

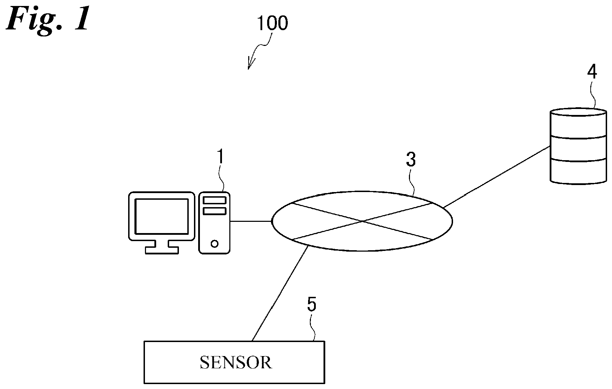

Fig. 1 is a schematic diagram illustrating an example of a biometric information computing system 100 in the first embodiment.

The biometric information computing system 100 is used for evaluating biometric information of a user. The biometric information computing system 100 obtains a plurality of evaluation data based on a pulse wave of the user, and generates an evaluation result including the biometric information to the evaluation data. That is, the plurality of evaluation data are obtained from one pulse wave of the user. Therefore, in obtaining each of the evaluation data, a variation caused by a measurement condition of the pulse wave can be eliminated. For example, based on the feature of one evaluation data, a process condition of another evaluation data may be specified. Additionally, from a plurality of evaluation data, mutually different evaluation results may be generated. In any case, since the evaluation is performed with one pulse wave of the user, the influence of the measuring condition can be suppressed. Accordingly, the accuracy improvement in the evaluation of biometric information can be attempted.

Especially, the biometric information computing system 100 according to the embodiment generates a plurality of evaluation results including mutually different kinds of biometric information from a plurality of evaluation data based on the pulse wave of the user. That is, based on one pulse wave measured from a user, the generation of the evaluation result including a plurality of kinds of biometric information can be achieved. The "biometric information" means, for example, a feature of blood, a feature of a body, and the like, which can be estimated from a pulse wave.

As the biometric information, for example, a feature of blood carbon dioxide is used, and additionally, for example, a blood glucose level, a blood pressure, an oxygen saturation, a lactate level, a pulse rate, a respiratory rate, a stress level, a vascular age, a degree of diabetes, and the like are used. The "feature of blood carbon dioxide" means a degree of carbon dioxide contained in user's blood. As the feature of blood carbon dioxide, for example, a value of blood carbon dioxide partial pressure (PaCO₂) is used, and additionally, a dissolved concentration of blood carbon dioxide or a concentration of bicarbonate (HCO₃⁻) contained in blood may be used, or a value considering pH of blood may be used corresponding to a situation.

For example, as illustrated in Fig. 1, the biometric information computing system 100 includes a biometric information computing device 1, and for example, may include at least any of a sensor 5 and a server 4. The biometric information computing device 1 is connected to the sensor 5 and the server 4 via, for example, a communications network 3.

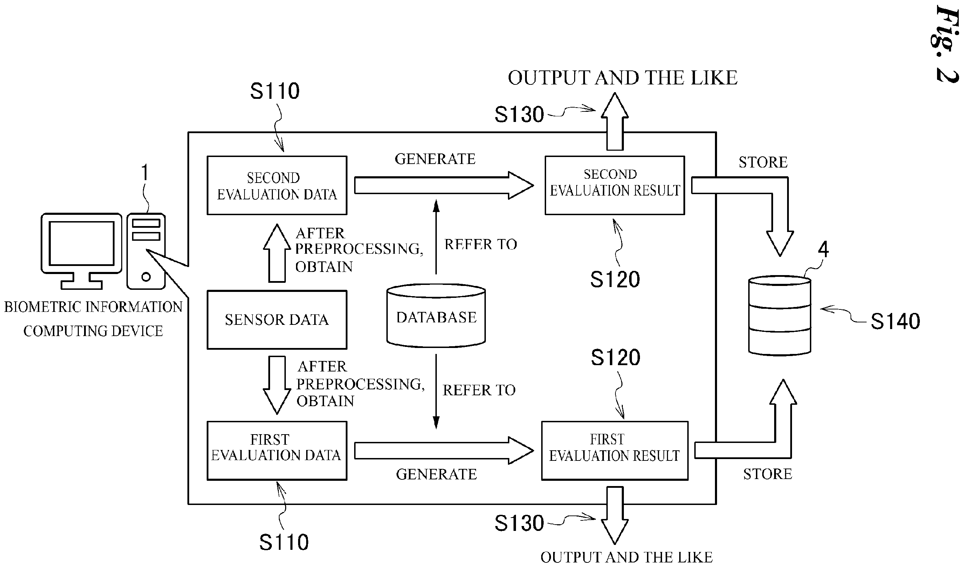

In the biometric information computing system 100, for example, as illustrated in Fig. 2A, the biometric information computing device 1 obtains sensor data generated by the sensor 5 or the like. The sensor data means data including a feature of one pulse wave of the user measured in a specific period. Then, the biometric information computing device 1 performs preprocessing such as a filter process on the obtained one sensor data, and obtains a plurality of evaluation data (for example, first evaluation data and second evaluation data). That is, the biometric information computing device 1 performs mutually different types of preprocessing to the one sensor data in obtaining the respective evaluation data.

The biometric information computing device 1 refers to a database, and calculates mutually different kinds of biometric information (for example, first biometric information and second biometric information) to the respective evaluation data. Then, the biometric information computing device 1 generates a plurality of evaluation results (for example, first evaluation result and second evaluation result) including mutually different kinds of biometric information. That is, a plurality of pieces of biometric information is calculated based on one pulse wave of the user. Therefore, when each piece of the biometric information is calculated, the variation caused by the measurement condition of the pulse wave can be eliminated. Accordingly, the accuracy improvement in the evaluation of the biometric information can be attempted.

Here, the biometric information computing device 1 refers to the database in the calculation of the biometric information to the respective evaluation data. The database stores classification information generated using a plurality of training data.

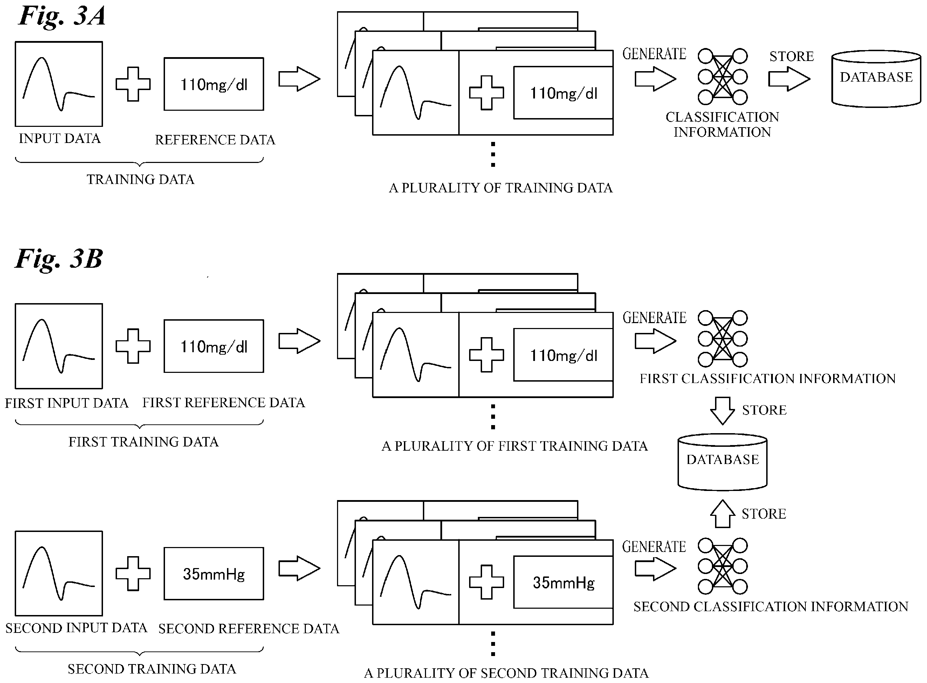

For example, as illustrated in Fig. 3A, the classification information is generated using a plurality of training data each of which is a pair of input data based on a training pulse wave obtained in the past and reference data including biometric information associated with the input data. Therefore, in the calculation of the biometric information, each evaluation result can be quantitatively generated based on the association of the feature of the pulse wave proven in the past with the biometric information. This allows suppressing the variation in the evaluation due to a subjective view of the user or the like.

For example, as illustrated in Fig. 3B, the classification information may include a plurality of pieces of classification information (for example, first classification information and second classification information) generated using mutually different kinds of training data. In this case, each of the evaluation results can be generated with reference to optimal classification information corresponding to the kind of each evaluation data.

The biometric information computing device 1 stores the plurality of generated evaluation results in the server 4 or the like. Accordingly, the secondary use of the respective evaluation results can be easily achieved.

The biometric information computing device 1 outputs the respective generated evaluation results to, for example, a display or the like. Accordingly, the user can obtain the plurality of generated evaluation results.

In the biometric information computing system 100, for example, the plurality of evaluation data may be obtained from the sensor 5 or the like. In this case, the preprocessing of obtaining the plurality of evaluation data from the sensor data is performed by the sensor 5 or the like.

### <Sensor Data>

The sensor data includes data indicating the feature of the user's pulse wave, and may include, for example, data (noise) indicating a feature other than the pulse wave. The sensor data is data indicating an amplitude to a measurement time, and by performing a filter process depending on the usage or the generation condition of the sensor data, data corresponding to an acceleration pulse wave, a velocity pulse wave, or the like can be obtained from the sensor data.

The sensor data can be generated with a publicly known sensor, such as a strain sensor, a gyro sensor, a photoplethysmogram (PPG) sensor, and a pressure sensor. The sensor data may be a digital signal, and additionally, for example, an analog signal. The measurement time in generating the sensor data is a measurement time by, for example, 1 to 20 cycles of the pulse wave, and can be appropriately set depending on the condition such as a processing method of the sensor data and a data communication method.

### <Evaluation Data>

The evaluation data indicates data for calculating the biometric information. The evaluation data indicates, for example, data corresponding to the acceleration pulse wave based on the user's pulse wave, and indicates an amplitude to a specific cycle (for example, one cycle).

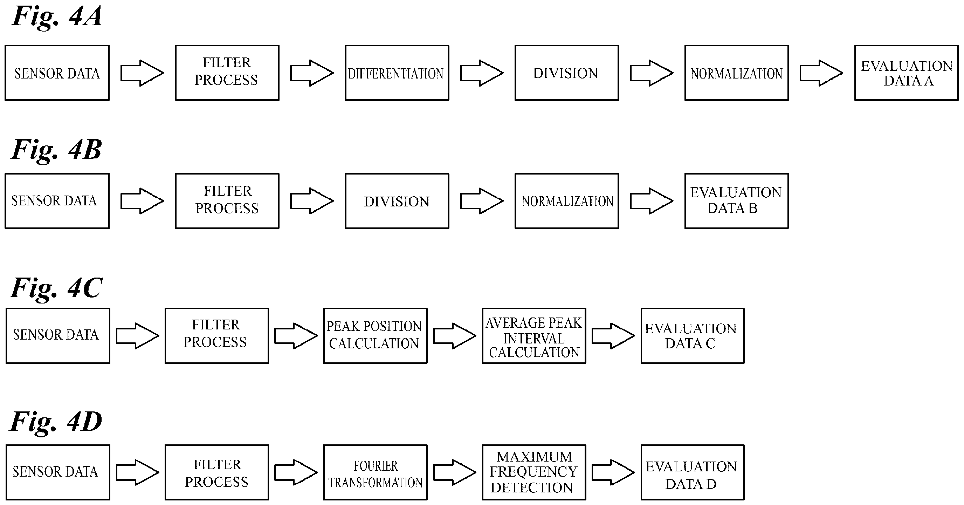

The evaluation data is obtained through performing a process (preprocessing) to the sensor data by the biometric information computing device 1 or the like. For example, as illustrated in Fig. 4A to Fig. 4D, by performing a plurality of processes on the sensor data, the evaluation data can be obtained. Details of the respective processes will be described later.

### <Database>

The database is mainly used for generating the evaluation result for the evaluation data. The database stores one or more pieces of the classification information, and additionally, may store, for example, the plurality of training data used for generating the classification information.

The classification information is a function indicating, for example, a correlation between preliminarily obtained past evaluation data (input data) and reference data including biometric information. The classification information indicates a calibration model generated based on a result of an analysis performed by, for example, a regression analysis having the input data as an explanatory variable and the reference data as an objective variable. For the classification information, for example, the calibration model can be regularly updated, and additionally, a plurality of calibration models generated for each piece of attribute information such as a gender, an age, and an exercise content of the user may be included.

As a method of the regression analysis used in the generation of the classification information, for example, PLS (Partial Least Squares) regression analysis, a regression analysis using SIMCA (Soft Independent Modeling of Class Analogy) method in which a principal component analysis is performed for each class to obtain a principal component model, or the like can be used.

The classification information may include, for example, a trained model generated through machine learning using a plurality of training data. The trained model indicates, for example, a neural network model such as CNN (Convolutional Neural Network), and additionally, indicates SVM (Support vector machine) or the like. As the machine learning, for example, deep learning can be used.

As the input data, data of the same kind as the evaluation data is used, and for example, past evaluation data in which the corresponding biometric information is clear is indicated. For example, the sensor 5 or the like is worn by an examinee, and sensor data (training sensor data) indicating the feature of the training pulse wave is generated. Then, by performing a process on the training sensor data, the input data can be obtained. The input data is obtained from the user of the biometric information computing system 100, and additionally, for example, may be obtained from another user. That is, the above-described examinee is a user of the biometric information computing system 100, and additionally, may be targeted to those other than the user, and may be a large number of specified or unspecified people.

The input data is preferably obtained, for example, with the contents similar to the type of the sensor 5 or the like used in obtaining the evaluation data, the generation condition of the sensor data, and the process condition to the sensor data. For example, by making the above-described three contents uniform, the accuracy in generating the biometric information can be tremendously improved.

The reference data includes the biometric information of the examinee measured using a measuring device or the like. For example, when the sensor 5 or the like is worn by the examinee to generate the training sensor data, by measuring the biometric information such as the feature of the blood carbon dioxide of the examinee, the reference data associated with the input data can be obtained. In this case, while a timing of measuring the biometric information is preferably simultaneous with a timing of generating the training sensor data, the timing of measuring the biometric information may be a timing, for example, before or after about one to 10 minutes.

The reference data is measured using a publicly known measuring device. For example, in the case of measuring the feature of the blood carbon dioxide concentration, as the measuring device, a device such as transcutaneous blood gas monitor TCM5 (manufactured by Radiometer Basel) is used. For example, when a blood lactate level is measured, as the measuring device, a publicly known device such as Lactate Pro 2 (manufactured by ARKRAY, Inc.) is used. For example, when an oxygen saturation is measured, as the measuring device, a publicly known device such as PULSOX-Neo (manufactured by KONICA MINOLTA, INC.) is used.

### <Biometric Information>

The biometric information calculated in the biometric information computing system 100 is calculated as data of the same kind as the reference data. The biometric information is calculated as data the same as or similar to the reference data with reference to the classification information. In the biometric information computing system 100, for example, each of a plurality of evaluation data is obtained along any time series, and a plurality of pieces of biometric information to the respective evaluation data are generated. In the biometric information computing system 100, for example, a plurality of evaluation data may be obtained for each of any timings, and a plurality of pieces of biometric information to the respective evaluation data may be calculated.

### <Evaluation Result>

The evaluation result includes the biometric information of the user calculated by the biometric information computing device 1. The evaluation result may include, for example, a comparison result with a preliminarily set threshold in addition to the biometric information. The evaluation result may include, for example, a value derived from the plurality of pieces of biometric information along time series. The evaluation result may include, for example, advice, such as "healthy," "exercise is needed," or "during anaerobic exercise," leading to the health condition or the health maintenance of the user, and information indicating the evaluation to the tendency of athletic ability. Accordingly, changes over time for each of the plurality of pieces of biometric information can be easily obtained. For example, by outputting the evaluation result, the biometric information of the user can be obtained.

### <Biometric Information Computing Device 1>

The biometric information computing device 1 indicates, for example, an electronic device such as a personal computer (PC), a mobile phone, a smartphone, a tablet terminal, and a wearable device, and indicates, for example, an electronic device communicatable via the communications network 3 based on the operation of the user. The biometric information computing device 1 may include the sensor 5. The following describes an example of a case where a PC is used as the biometric information computing device 1.

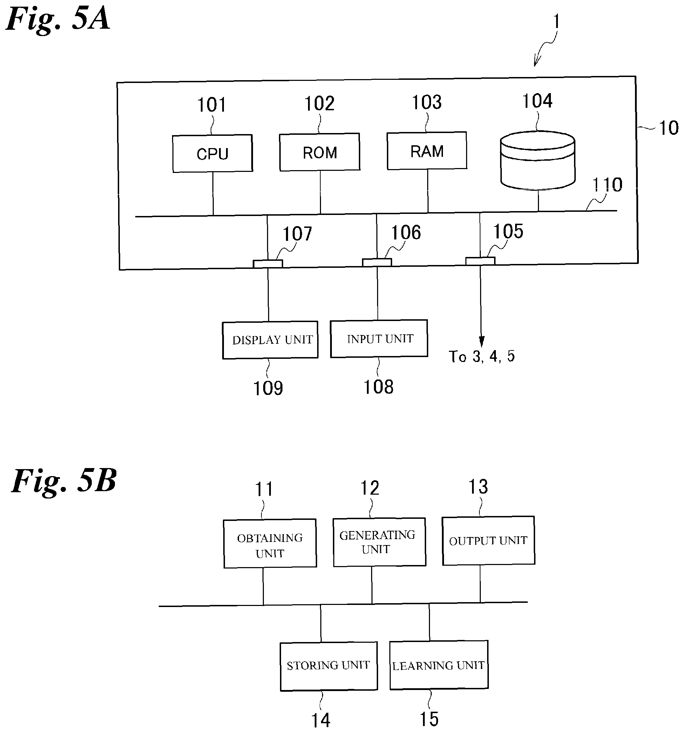

Fig. 5A is a schematic diagram illustrating an example of a configuration of the biometric information computing device 1, and Fig. 5B is a schematic diagram illustrating an example of functions of the biometric information computing device 1.

For example, as illustrated in Fig. 5A, the biometric information computing device 1 includes a housing 10, a CPU (Central Processing Unit) 101, a ROM (Read Only Memory) 102, a RAM (Random Access Memory) 103, a storage unit 104, and I/Fs 105 to 107. The configurations 101 to 107 are mutually connected via an internal bus 110.

The CPU 101 controls the entire biometric information computing device 1. The ROM 102 stores operation codes of the CPU 101. The RAM 103 is a work area used in the operation of the CPU 101. The storage unit 104 stores various kinds of information such as the database and the evaluation data. As the storage unit 104, for example, a data storage device such as an SSD (Solid State Drive) or the like is used in addition to an HDD (Hard Disk Drive). For example, the biometric information computing device 1 may include a not illustrated GPU (Graphics Processing Unit).

The I/F 105 is an interface for transmitting and receiving various kinds of information with the server 4, the sensor 5, and the like via the communications network 3 as necessary. The I/F 106 is an interface for transmitting and receiving the information with an input unit 108. As the input unit 108, for example, a keyboard is used, and the user or the like of the biometric information computing device 1 inputs various kinds of information, control commands of the biometric information computing device 1, or the like via the input unit 108. The I/F 107 is an interface for transmitting and receiving various kinds of information with a display unit 109. The display unit 109 displays various kinds of information, the evaluation result, or the like stored in the storage unit 104. As the display unit 109, a display is used, and for example, in a case of a touch panel type, the display unit 109 is provided integrally with the input unit 108.

Fig. 5B is a schematic diagram illustrating an example of functions of the biometric information computing device 1. The biometric information computing device 1 includes an obtaining unit 11, a generating unit 12, an output unit 13, and a storing unit 14, and may include, for example, a learning unit 15. The functions illustrated in Fig. 5B are each achieved by executing programs stored in the storage unit 104 or the like by the CPU 101 with the RAM 103 as the work area.

### < Obtaining Unit 11>

The obtaining unit 11 obtains a plurality of evaluation data based on the user's pulse wave. For example, after obtaining the sensor data from the sensor 5 or the like, the obtaining unit 11 performs a process on the sensor data to obtain the evaluation data. The obtaining unit 11 performs mutually different types of the processes, thereby obtaining a plurality of evaluation data to one sensor data.

For example, as illustrated in Fig. 4A, the obtaining unit 11 performs a filtering process (filter process) on the obtained sensor data. In the filter process, for example, a bandpass filter of 0.5 to 5.0 Hz is used. Accordingly, the obtaining unit 11 extracts data (pulse wave data) corresponding to the user's pulse wave. The pulse wave data indicates, for example, data corresponding to the velocity pulse wave. The pulse wave data may indicate data corresponding to, for example, the acceleration pulse wave or the plethysmogram, and can be appropriately set depending on the type or the usage of the sensor. The filter range of bandpass filter can be appropriately set depending on the usage.

The obtaining unit 11 performs, for example, a differential process on the pulse wave data. For example, when the differential process is performed to the pulse wave data corresponding to the velocity pulse wave, the obtaining unit 11 obtains data (differential data) corresponding to the acceleration pulse wave. In the differential process, a two time differentiation may be performed in addition to a one time differentiation.

The obtaining unit 11 performs, for example, a dividing process on the differential data. In the dividing process, for example, differential data corresponding to the acceleration pulse wave of a plurality of cycles is divided into data (divided data) corresponding to the acceleration pulse waves of respective cycles. Therefore, for example, by performing the differential process on one differential data, the obtaining unit 11 can obtain a plurality of divided data. In the dividing process, the differential data can be divided for each of any cycles (for example, positive number multiple of cycle) depending on the usage.

For example, in the dividing process, the data amounts of the divided respective divided data are mutually different in some cases. In this case, the obtaining unit 11 may identify the divided data with the least data amount, and may perform reduction of the data amount (trimming) to the other divided data. This allows making the data amounts of the respective divided data uniform, thus facilitating data comparison between the respective divided data.

Additionally, for example, a normalization process may be performed to a value corresponding to a time axis of the divided data. In the normalization process, for example, the normalization in which the minimum value of the value corresponding to the time axis is set to 0 and the maximum value is set to 1 is performed. Accordingly, the data comparison between the respective divided data is facilitated.

The obtaining unit 11 may provide the divided data by, for example, calculating a mean of a plurality of divided data to which the reduction of the data amount or the normalization has been performed.

The obtaining unit 11 performs the normalization process on the divided data. In the normalization process, data of being normalized (normalized data) is generated to a value corresponding to the amplitude. In the normalization process, for example, the normalization in which the lowest value of the amplitude is set to 0 and the highest value of the amplitude is set to 1 is performed. The obtaining unit 11 obtains, for example, the normalized data as evaluation data (for example, evaluation data A). In this case, as the evaluation data A, data corresponding to the acceleration pulse wave of the user is obtained.

The obtaining unit 11 sequentially performs the above-described respective processes, and additionally, for example, as illustrated in Fig. 4B, the obtaining unit 11 does not need to perform the differential process. In this case, as the evaluation data (for example, evaluation data B), data corresponding to the velocity pulse wave of the user is obtained.

The obtaining unit 11 may perform, for example, only a part of the above-described processes. In this case, the obtaining unit 11 may obtain any of the pulse wave data, the differential data, the divided data, the trimmed divided data, and the divided data in which the value corresponding to the time axis has been normalized as the evaluation data, and the setting can be appropriately made depending on the usage.

For example, as illustrated in Fig. 4C, the obtaining unit 11 may obtain evaluation data C appropriate for calculating the pulse rate as the biometric information.

In this case, the obtaining unit 11 performs the above-described filter process on the sensor data, thereby extracting the pulse wave data. Then, the obtaining unit 11 performs a peak position calculating process on the pulse wave data. In the peak position calculating process, a plurality of peaks (maximum values of the amplitude) included in the pulse wave data are detected, and the order of sampling (corresponding to a time after the measurement start) is specified. Accordingly, the obtaining unit 11 obtains peak position data included in the pulse wave data.

Then, the obtaining unit 11 performs an average peak interval calculating process on the peak position data. In the average peak interval calculating process, intervals of the peaks included in the peak position data (difference between adjacent peaks in the sampling order) are calculated, and for example, the average value of the peak interval is calculated. Subsequently, the obtaining unit 11 divides the peak interval or the average value of the peak interval by the sampling rate of the sensor data, thus obtaining data indicating the peak interval corresponding to the number of seconds as the evaluation data (for example, evaluation data C).

For example, as illustrated in Fig. 4D, the obtaining unit 11 may obtain evaluation data D appropriate for calculating the respiratory rate as the biometric information.

In this case, the obtaining unit 11 performs the above-described filter process on the sensor data, thereby extracting the pulse wave data. Then, the obtaining unit 11 performs Fourier transform processing to the pulse wave data. In the Fourier transform processing, for example, the pulse wave data indicating the sampling time to the amplitude is transformed to frequency data indicating the frequency to the intensity. Accordingly, the obtaining unit 11 obtains frequency data to the pulse wave data.

Then, the obtaining unit 11 performs a maximum frequency detecting process on the frequency data. In the maximum frequency detecting process, in the frequency data, the frequency with the maximum intensity in a range from 0.15 to 0.35 Hz is identified. Accordingly, the obtaining unit 11 obtains the value of the identified frequency as evaluation data D.

### <Generating Unit 12>

The generating unit 12 generates the evaluation result for the evaluation data with reference to the database. The generating unit 12 refers to, for example, the classification information stored in the database, calculates the biometric information to the evaluation data, and generates it as the evaluation result. The generating unit 12 generates a plurality of the evaluation results to the respective mutually different plurality of evaluation data.

The generating unit 12 may generate, for example, a plurality of evaluation data to a plurality of evaluation data with reference to the same classification information. In this case, for example, by obtaining a plurality of evaluation data using the mutually different kinds of preprocessing, mutually different kinds of biometric information can be calculated even when the same classification information is used. This eliminates the need for generating the classification information for each of the evaluation data, thus allowing reducing the volume of data in the database.

The generating unit 12 may calculate, for example, a plurality of pieces of biometric information to the plurality of evaluation data by referring to the mutually different classification information. In this case, by referring to optimal classification information corresponding to the kinds of the respective evaluation data, the respective evaluation results can be generated.

The generating unit 12 may generate the evaluation result converted into a format in which the user can understand the biometric information, for example, using a display format preliminarily stored in the storage unit 104 or the like.

### <Output Unit 13>

The output unit 13 outputs a plurality of evaluation results. The output unit 13 outputs the plurality of evaluation results to the display unit 109, and additionally, may output the plurality of evaluation results to, for example, the sensor 5 or the like.

### <Storing Unit 14>

The storing unit 14 retrieves various kinds of data such as a database stored in the storage unit 104 as necessary. The storing unit 14 stores the various kinds of data obtained or generated by the configurations 11 to 13, and 15 in the storage unit 104 as necessary.

The storing unit 14 may, for example, store the sensor data associated with the plurality of evaluation results in the storage unit 104. For example, the storing unit 14 may also store the plurality of evaluation data generated using the sensor data in the storage unit 104.

### <Learning Unit 15>

The learning unit 15 generates the classification information, for example, using a plurality of training data. The learning unit 15 may, for example, obtain new training data and update the existing classification information.

### <Communications Network 3>

The communications network 3 is a publicly known Internet network or the like in which the biometric information computing device 1, the server 4, and the sensor 5 are connected via a communication line. The communications network 3 may be established by a LAN (Local Area Network) or the like when the biometric information computing system 100 is operated in a certain narrow area. The communications network 3 may be established by what is called an optical fiber communications network. The communications network 3 is not limited to a wired communication network, may be achieved by a wireless communications network, and can be appropriately set depending on the usage.

### <Server 4>

The server 4 stores and accumulates various kinds of information such as the evaluation result transmitted via the communications network 3. The server 4 transmits the accumulated information to the biometric information computing device 1 via the communications network 3 based on the request from the biometric information computing device 1.

The server 4 may be connected to, for example, a plurality of biometric information computing devices 1, obtain the various kinds of information such as the evaluation result from each of the biometric information computing devices 1, and collectively store the various kinds of information. The server 4 may include at least a part of the functions among the above-described functions included in the biometric information computing device 1. The server 4 may store the above-described database or the like stored in the biometric information computing device 1.

<Sensor 5>

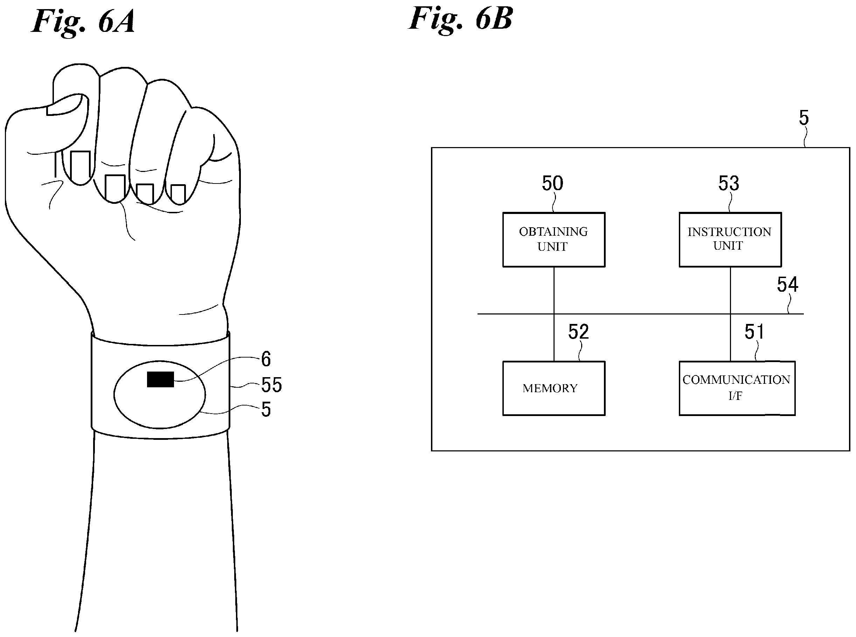

The sensor 5 generates the sensor data. For example, as illustrated in Fig. 6A, the sensor 5 includes a detecting unit 6. The sensor 5 is worn at a position at which the user's pulse wave can be detected via the detecting unit 6, and for example, fixed to a wristband 55.

As the detecting unit 6, a publicly known detecting device that can detect the user's pulse wave is used. As the detecting unit 6, for example, at least any of a strain sensor such as a fiber bragg grating (FBG) sensor, a gyro sensor, one or more electrodes for measuring a pulse wave signal, a photoplethysmogram (PPG) sensor, a pressure sensor, and a photo-detection module is used. For example, a plurality of the detecting units 6 may be provided.

The sensor 5 may be embedded in clothing. The user wearing the sensor 5 may be not only a human but also a pet such as a dog and a cat, and for example, may be a domestic animal such as a cow and a pig, a farmed fish, or the like.

For example, as illustrated in Fig. 6B, the sensor 5 includes an obtaining unit 50, a communication I/F 51, a memory 52, and an instruction unit 53, and the configurations are mutually connected by an internal bus 54.

The obtaining unit 50 measures the user's pulse wave via the detecting unit 6, and generates the sensor data. The obtaining unit 50 transmits, for example, the generated sensor data to the communication I/F 51 or the memory 52.

The communication I/F 51 transmits the various kinds of data such as sensor data to the biometric information computing device 1 and the server 4 via the communications network 3. The communication I/F 51 includes a line control circuit for connecting to the communications network 3, a signal conversion circuit for performing a data communication with the biometric information computing device 1 and the server 4, and the like. The communication I/F 51 performs conversion processing to various kinds of instructions from the internal bus 54 and delivers these to the communications network 3 side, and when data from the communications network 3 is received, the communication I/F 51 performs predetermined conversion processing to the data, and transmits it to the internal bus 54.

The memory 52 stores various kinds of data such as sensor data transmitted from the obtaining unit 50. For example, the memory 52 receives an instruction from another terminal device connected via the communications network 3, thereby transmitting the stored various kinds of data such as sensor data to the communication I/F 51.

The instruction unit 53 includes an operating button, a keyboard, or the like for obtaining the sensor data, and for example, includes a processor such as a CPU. When an instruction to obtain the sensor data is accepted, the instruction unit 53 notifies the obtaining unit 50 of it. The obtaining unit 50 that has received the notification obtains the sensor data. For example, as illustrated in Fig. 4A to Fig. 4D, the instruction unit 53 may perform a process for obtaining a plurality of evaluation data from the sensor data.

Here, as an example of obtaining the sensor data, a case of using an FBG sensor will be described.

The FBG sensor is one in which diffraction grating structures are formed at predetermined intervals in one optical fiber. The FBG sensor has a feature of, for example, a sensor part length of 10 mm, a wavelength resolution of ±0.1 pm, a wavelength range of 1550 ± 0.5 nm, a fiber diameter of 145 µm, and a core diameter of 10.5 µm. The measurement can be performed with the FBG sensor as the above-described detecting unit 6 in contact with the user's skin.

For example, as a light source used for the optical fiber, an ASE (Amplified Spontaneous Emission) light source with the wavelength range of 1525 to 1570 nm is used. An emitted light from the light source enters the FBG sensor via a circulator. A reflected light from the FBG sensor is guided to a Mach-Zehnder interferometer via the circulator, and an output light from the Mach-Zehnder interferometer is detected by an optical detector. The Mach-Zehnder interferometer is one for creating an interference light by splitting an optical path into two optical paths with an optical path difference by a beam splitter and superimposing the two optical paths into one again by the beam splitter. To provide the optical path difference, for example, the length of one optical fiber may be lengthened. Since interference fringes are produced in a coherent light corresponding to the optical path difference, by measuring the pattern of the interference fringe, the strain change in the FBG sensor, that is, the pulse wave can be detected. The obtaining unit 50 generates the sensor data based on the detected pulse wave. Accordingly, the sensor data is obtained.

The optical fiber sensor system that detects the waveform of the pulse wave by detecting the strain amount of the FBG sensor includes, in addition to the light source whose light is entered in the FBG sensor, an optical system such as an ASE light source with a wide bandwidth, a circulator, a Mach-Zehnder interferometer, and a beam splitter, a light receiving sensor included in the optical detector, and an analysis method for analyzing a wavelength shift amount. The optical fiber sensor system can be used by selecting the light source and bandwidth light depending on the characteristics of the FBG sensor to be used, and for the analysis method such as a wave detection method, various kinds of methods are employable.

### <Data Structure>

For example, a data structure including a plurality of evaluation results (for example, a first evaluation result and a second evaluation result) generated by the above-described biometric information computing system 100 is stored in the server 4 or the storage unit 104. The data structure is used in the above-described biometric information computing device 1 (computer including the display unit 109, the CPU 101 (control unit), and the storage unit 104). The data structure including the plurality of evaluation results is used, for example, when the generating unit 12 controlled by the CPU 101 generates a comprehensive evaluation result based on each of the evaluation results. The comprehensive evaluation result will be described later.

### (First Embodiment: Operation of Biometric Information Computing System 100)

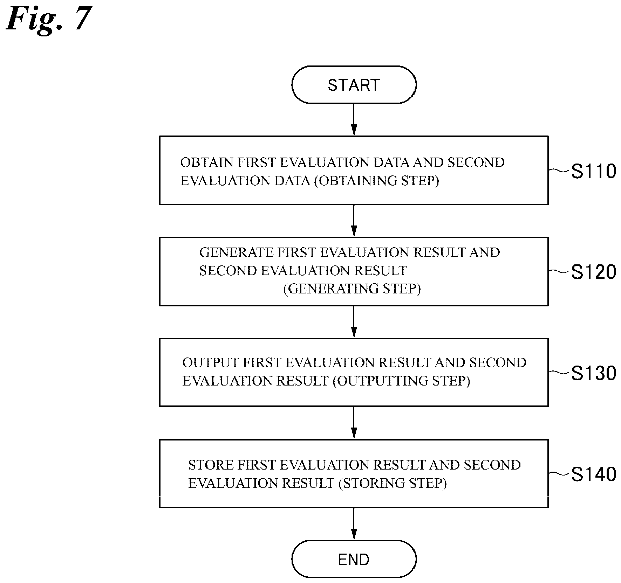

Next, an example of the operation of the biometric information computing system 100 according to the embodiment will be described. Fig. 7 is a flowchart illustrating an example of the operation of the biometric information computing system 100 in this embodiment.

The biometric information computing system 100 is executed, for example, via a biometric information computing program installed in the biometric information computing device 1. That is, the user operates the biometric information computing device 1 or the sensor 5, thereby being able to obtain a plurality of evaluation results including the biometric information of the user from the sensor data through the biometric information computing program installed in the biometric information computing device 1.

The operation of the biometric information computing system 100 includes an obtaining step S 110, a generating step S 120, and a storing step S 140, and may include, for example, an outputting step S130.

### <Obtaining Step S110>

The obtaining step S 110 obtains a plurality of evaluation data based on the user's pulse wave. For example, the obtaining unit 50 of the sensor 5 measures the user's pulse wave via the detecting unit 6 to generate the sensor data. The obtaining unit 50 transmits the sensor data to the biometric information computing device 1 via the communication I/F 51 and the communications network 3. The obtaining unit 11 of the biometric information computing device 1 receives the sensor data from the sensor 5.

The obtaining unit 11 performs, for example, the processes illustrated in Figs. 4A and 4B to the sensor data, thereby obtaining the first evaluation data and the second evaluation data. The obtaining unit 11 stores each of the obtained evaluation data in the storage unit 104, for example, via the storing unit 14. The condition such as a frequency of obtaining the sensor data from the sensor 5 by the obtaining unit 11 can be appropriately set depending on the usage. For example, the obtaining unit 11 obtains each of the evaluation data in a preliminarily set cycle.

### <Generating Step S120>

Next, the generating step S120 refers to the database to generate a plurality of evaluation results including the biometric information to the respective evaluation data. For example, the generating unit 12 refers to the classification information, thereby calculating a value of the blood carbon dioxide partial pressure to the first evaluation data as first biometric information, and calculating a blood glucose level to the second evaluation data as second biometric information. The generating unit 12 generates the first evaluation result including the first biometric information and the second evaluation result including the second biometric information.

For example, the generating unit 12 generates the first evaluation result for the first evaluation data by referring to the first classification information, and generates the second evaluation result for the second evaluation data by referring to the second classification information. In this respect, the first classification information and the second classification information are included in the classification information, and generated using mutually different kinds of training data.

The generating unit 12 stores the respective generated evaluation results in the storage unit 104, for example, via the storing unit 14. As the respective evaluation results, in addition to specific values, for example, error ranges (for example, "xx ± 2 mmHg") may be calculated.

For example, when the pulse rate is calculated as the biometric information, as the evaluation data, for example, the evaluation data C illustrated in Fig. 4C is used, and the generating unit 12 refers to pulse rate classification information included in the classification information. The pulse rate classification information indicates, for example, a function of dividing 60 [second] by a peak interval. Therefore, the generating unit 12 can calculate, for example, the pulse rate (= 71 [bpm]) relative to the evaluation data C (peak interval = 0.85 [second]). Accordingly, the generating unit 12 can generate the evaluation result including the biometric information indicating the pulse rate.

For example, when the respiratory rate is calculated as the biometric information, as the evaluation data, for example, the evaluation data D illustrated in Fig. 4D is used, and the generating unit 12 refers to respiratory rate classification information included in the classification information. The respiratory rate classification information indicates, for example, a function of multiplying a specified frequency by 60 [second]. Therefore, the generating unit 12 can calculate, for example, the respiratory rate (=13.5 [bpm]) relative to the evaluation data D (specified frequency = 0.225 Hz). Accordingly, the generating unit 12 can generate the evaluation result including the biometric information indicating the respiratory rate.

### <Outputting Step S130>

Next, for example, the outputting step S130 may output a plurality of evaluation results. For example, the output unit 13 outputs the first evaluation result and the second evaluation result to the display unit 109.

### <Storing Step S140>

Next, the storing step S140 stores the first evaluation result and the second evaluation result. For example, the storing unit 14 stores the first evaluation result and the second evaluation result in the storage unit 104. For example, the output unit 13 may output the first evaluation result and the second evaluation result to the server 4 via the communications network 3, and store the first evaluation result and the second evaluation result. For example, the storing step S140 may be performed before the outputting step S130.

The storing step S140 may store, for example, the sensor data associated with a plurality of evaluation results. In this case, the sensor data is used for generating a plurality of evaluation data, and indicates the feature of the user's pulse wave.

Accordingly, the operation of the biometric information computing system 100 ends. The frequency and the order of executing the steps can be appropriately set depending on the usage.

In the biometric information computing system 100, for example, the above-described steps S110, S120 are performed by the biometric information computing device 1, and additionally, at least a part may be performed by the server 4. In this case, each of the above-described steps S110, S120 includes a process for transmitting and receiving the various kinds of information via the communications network 3 by the server 4. The communication between the biometric information computing device 1 and the server 4 can be achieved using a publicly known technique.

For example, in the obtaining step S 110, the plurality of evaluation data may be obtained by the server 4. In this case, the obtaining unit included in the server 4 obtains the plurality of evaluation data transmitted from the biometric information computing device 1 via the communications network 3.

The obtaining unit included in the server 4 may obtain the plurality of evaluation data, for example, by obtaining the sensor data transmitted from the biometric information computing device 1 or the sensor 5 and performing the above-described preprocessing. In this case, in the biometric information computing device 1, a load of performing the above-described preprocessing can be reduced.

For example, in the generating step S 120, a plurality of evaluation results to the respective evaluation data may be generated by the server 4. In this case, the generating unit included in the server 4 refers to the database stored in the server 4, thereby generating a plurality of evaluation results. In this case, in the biometric information computing device 1, a load of performing the above-described process of generating a plurality of evaluation results can be reduced.

When the generating step S120 is performed by the server 4, in the outputting step S130, for example, the output unit included in the server 4 transmits the plurality of evaluation results to the biometric information computing device 1 or the like via the communications network 3. In this case, the output unit 13 of the biometric information computing device 1 outputs the received plurality of evaluation results to the display unit 109.

As described above, the steps S110 to S140 in the biometric information computing system 100 can be performed by both of the biometric information computing device 1 and the server 4. Especially, by performing the obtaining step S110 and the generating step S120 by the server 4, for example, the reduction of the load on the biometric information computing device 1 can be attempted. Since the same applies to the embodiments described later, the explanations will be omitted.

Here, in the evaluation of the biometric information of the user, it is desired to evaluate a plurality of pieces of biometric information at once. In this respect, in the prior art, it is premised that two plethysmograms are used in the measurement of the blood carbon dioxide saturation, hemoglobin, and the like among the biometric information. Therefore, the variation of data caused by the measurement conditions of the respective plethysmograms significantly affects the estimation accuracy in some cases. When a plurality of pieces of biometric information are estimated using the prior art, it is necessary to obtain a plurality of plethysmograms corresponding to the number and the kinds of the biometric information. Therefore, it is concerned that in proportion to the number of plethysmograms necessary for estimating the plurality of pieces of biometric information, the estimation accuracy is reduced due to the variation for each plethysmogram.

In contrast, according to this embodiment, the obtaining unit 11 obtains the first evaluation data and the second evaluation data based on the user's pulse wave. Additionally, the generating unit 12 generates each of the first evaluation result including the first biometric information for the first evaluation data and the second evaluation result including the second biometric information, which is a different kind from the first biometric information, to the second evaluation data. That is, the first biometric information and the second biometric information are calculated based on one pulse wave of the user. Therefore, in the calculation of the respective pieces of the biometric information, the variation caused by the measurement condition of the pulse wave can be eliminated. Accordingly, the accuracy improvement in the evaluation of the biometric information can be attempted.

According to this embodiment, the generating unit 12 generates the first evaluation result and the second evaluation result with reference to the database. The database stores the classification information calculated using a plurality of training data. Therefore, in generating the evaluation results, each of the evaluation results can be quantitatively generated based on the association of the feature of the pulse wave proven in the past with the biometric information. Accordingly, the variation in the evaluation due to a subjective view of the user or the like can be suppressed.

According to this embodiment, the obtaining unit 11 obtains the first evaluation data and the second evaluation data by performing the mutually different kinds of the processes on one pulse wave data corresponding to any of the velocity pulse wave and the acceleration pulse wave based on the user's pulse wave. Therefore, in the comparison with the case of using the photoelectric plethysmogram, the evaluation using the training data and the respective evaluation data in which the influence of noise data is suppressed can be performed. Accordingly, the evaluation with high accuracy can be achieved.

According to this embodiment, the classification information includes the first classification information and the second classification information generated using the different kinds of the training data. Therefore, each of the evaluation results can be generated with reference to the optimal classification information depending on the kind of the evaluation data. Accordingly, the further improvement of the accuracy in the evaluation of the biometric information can be attempted.

According to this embodiment, the classification information is a calibration model obtained using the PLS regression analysis having the input data as the explanatory variable and the reference data as the objective variable. Therefore, in the comparison with the case of calculating the classification information using machine learning or the like, the number of the training data can be substantially reduced, and the calibration model can be easily updated. Accordingly, the facilitation of establishing and updating the biometric information computing system 100 can be attempted.

According to this embodiment, the first biometric information indicates the blood glucose level, and the second biometric information indicates at least any of the blood pressure, the pulse rate, the respiratory rate, the feature of the blood carbon dioxide concentration, the lactate level, and the oxygen saturation. Therefore, since the invasive measurement method is not necessary compared with the conventional measurement method, each piece of the information can be easily obtained. Accordingly, the load on the user can be substantially reduced.

According to this embodiment, the storing unit 14 or the server 4 includes storing the sensor data associated with the first evaluation result and the second evaluation result. Therefore, in updating or additionally generating the classification information, the training data can be easily prepared. Accordingly, the maintenance of the biometric information computing system 100 can be easily achieved.

According to this embodiment, the server 4 generates the first evaluation result and the second evaluation result by the generating unit included in the server 4. The biometric information computing device 1 receives the first evaluation result and the second evaluation result from the server 4, and displays them. Therefore, to the biometric information computing device 1, the loads of generating the respective evaluation results can be reduced. Accordingly, the convenience of the biometric information computing device 1 can be improved. Additionally, the need for storing the database in the biometric information computing device 1 is eliminated. Accordingly, the data storage capacity of the biometric information computing device 1 can be substantially reduced. Additionally, by storing the database in the server 4, the evaluation result generated with reference to one piece of the classification information can be output to a plurality of biometric information computing devices 1. Accordingly, the huge amount of time and money for updating the database for each biometric information computing device 1 in association with the maintenance such as an update of the database can be reduced.

According to this embodiment, the server 4 can store the first evaluation result and the second evaluation result after attempting the improvement of accuracy in the evaluation of the biometric information.

According to this embodiment, the data structure includes the first evaluation result and the second evaluation result after attempting the improvement of accuracy in the evaluation of the biometric information, and can be used for generating the comprehensive evaluation result.

### (Second Embodiment: Biometric Information Computing System 100)

Next, an example of a biometric information computing system 100 in the second embodiment will be described. The difference between the above-described embodiment and the second embodiment is that additional information is used. For the contents similar to the above-described embodiment, the explanation will be omitted.

The biometric information computing system 100 according to this embodiment includes, for example, a comprehensive evaluation step S150. In the biometric information computing system 100, for example, the comprehensive evaluation step S 150 is performed after the above-described generating step S 120, and the storing step S 140 is performed after the comprehensive evaluation step S 150.

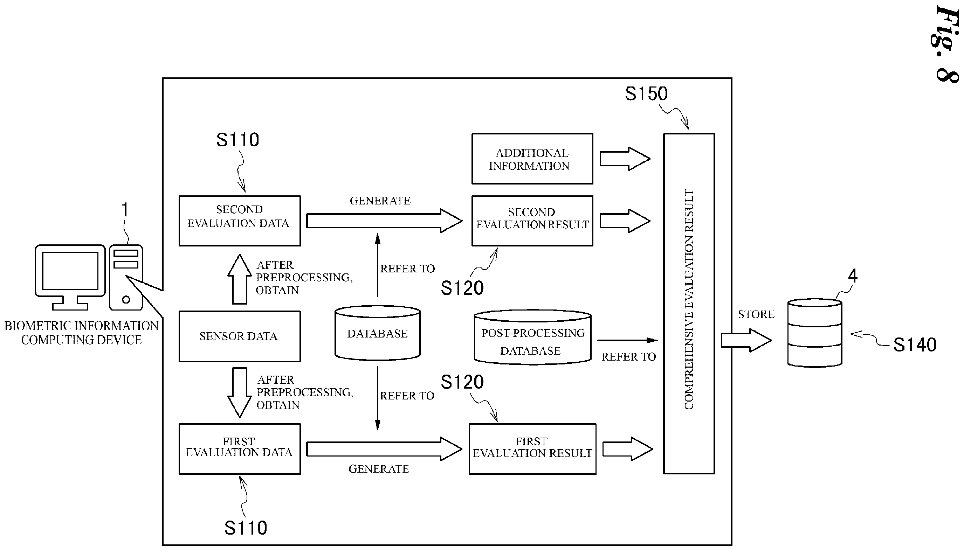

For example, as illustrated in Fig. 8, the comprehensive evaluation step S150 obtains the additional information, and generates a comprehensive evaluation result based on a plurality of evaluation results (for example, first evaluation result and second evaluation result) and the additional information. The comprehensive evaluation step S150 can be executed by, for example, a comprehensive evaluation unit included in the generating unit 12.

The additional information indicates the feature of the user, and indicates, for example, information different from the above-described biometric information. In this case, as the additional information, for example, attribute information such as a gender and an age of the user is used, and additionally, information specifying the health condition or the like of the user, such as a diagnostic result and an exercise quantity, may be used. The additional information includes, for example, information on the user, such as a case history, lifestyle habits, medication information, a degree of arteriosclerosis, and genetic information, and additionally, for example, information possibly relating to human body, such as temperature and humidity of a surrounding environment and an acceleration measured by an acceleration sensor attached to the sensor 5, may be included. The additional information is, for example, input via the input unit 108 or the like by the user, and obtained by a comprehensive obtaining unit or the like.

The comprehensive evaluation result indicates the result of comprehensively evaluating the feature of the user. The comprehensive evaluation result indicates, for example, the result of performing a correction process or the like based on the additional information to the biometric information included in each evaluation result. For example, when the user's age is used as the additional information, a comparison result of a reference value preliminarily set for each age with each of the evaluation results is generated as the comprehensive evaluation result.

Additionally, as the comprehensive evaluation result, for example, character strings indicating the features for each user, such as "blood glucose level is high," "blood pressure is high," "athletic ability is high," and "reducing exercise quantity is recommended," are used, and further, for example, a value such as a difference from any reference value and a deviation value may be used.

The comprehensive evaluation result indicates, for example, estimated insurance information including an insurance premium. The estimated insurance information includes, for example, a value indicating the insurance premium estimated based on each evaluation result, and additionally, for example, may include a character string indicating a type of the insurance or the like. The estimated insurance premium is calculated based on, for example, actuarial science.

The comprehensive evaluation unit generates the comprehensive evaluation result by referring to, for example, the data format that is preliminarily stored in the storage unit 104 or the like and recognizable by the user. The comprehensive evaluation unit may generate the appropriate comprehensive evaluation result for the plurality of evaluation results and the additional information by referring to, for example, a post-processing database. The post-processing database is stored in, for example, the storage unit 104.

In the post-processing database, for example, similarly to the above-described database, post-processing classification information for generating the comprehensive evaluation result for the plurality of evaluation results and the additional information may be stored. In the post-processing database, one or more pieces of the post-processing classification information are stored, and additionally, for example, a plurality of post-processing training data used for generating the post-processing classification information may be stored.

The post-processing classification information is, for example, a function indicating a correlation between a plurality of past evaluation results and past additional information (post-processing input data), which are preliminarily obtained, and post-processing reference data associated with the post-processing input data. The post-processing reference data indicates the result of comprehensively evaluating the feature of the user. The post-processing classification information is generated using a plurality of post-processing training data each of which is a pair of the post-processing input data and the post-processing reference data.

The post-processing classification information indicates a calibration model generated based on a result of an analysis performed by, for example, the above-described regression analysis having the post-processing input data as an explanatory variable and the post-processing reference data as an objective variable. For the post-processing classification information, for example, the calibration model (post-processing calibration model) can be regularly updated, and additionally, for example, may be generated for each piece of the additional information. The post-processing classification information may include, similarly to the above-described classification information, for example, a trained model (post-processing trained model) generated by machine learning using a plurality of post-processing training data.

The storing step S140 stores the comprehensive evaluation result. In the storing step S 140, by executing the process similar to that in the above-described embodiment, the comprehensive evaluation result is stored in at least any of the storage unit 104 and the server 4.

According to this embodiment, in addition to the effect of the above-described embodiment, the comprehensive evaluation unit generates the comprehensive evaluation result by comprehensively evaluating the feature of the user based on the first evaluation result, the second evaluation result, and the additional information. Therefore, for each of the evaluation results, the evaluation considering the feature of the user can be achieved. Accordingly, the evaluation result appropriate for each user can be generated.

### (Second Embodiment: Modification of Biometric Information Computing System 100)

Next, a modification of the biometric information computing system 100 in the second embodiment will be described. The difference between the above-described second embodiment and the modification is that the above-described additional information is obtained in the generating step S 120. For the contents similar to the above-described embodiment, the explanation will be omitted.

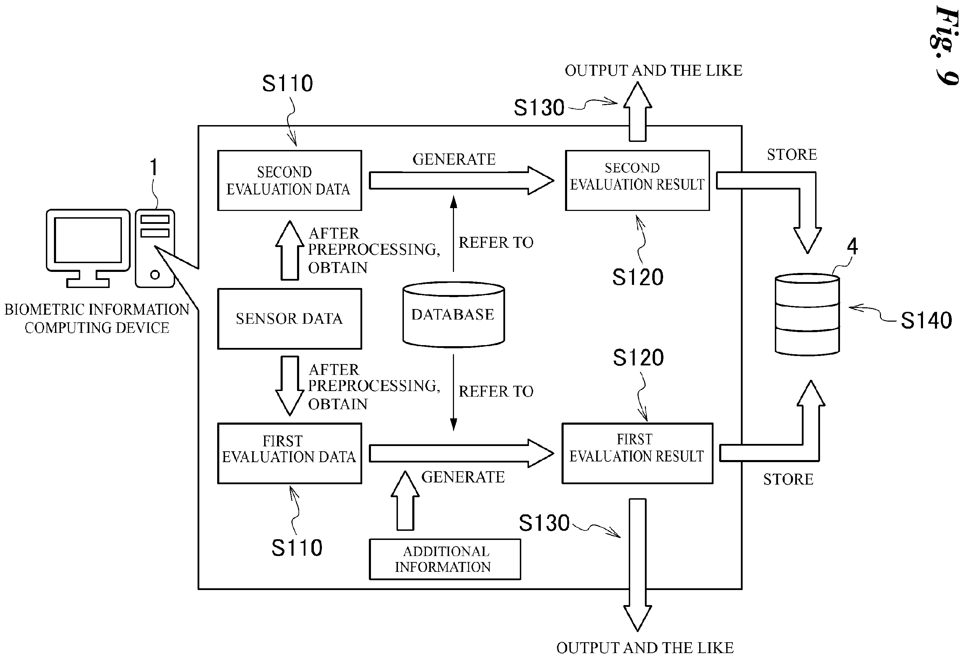

In this modification, for example, as illustrated in Fig. 9, the generating step S 120 includes obtaining the additional information and generating the first evaluation result based on the first evaluation data and the additional information. The additional information is similar to the above-described contents, and for example, input by the user via the input unit 108 or the like and obtained by the generating unit 12 or the like.

The generating unit 12 may determine the computing method to the first evaluation data, for example, corresponding to the contents of the additional information. In this case, functions or the like different between the kinds of the additional information are included in the classification information. The generating unit 12 may generate the first evaluation result, for example, based on the information in which the first evaluation data is combined with the additional information.

According to this modification, the generating unit 12 includes obtaining the additional information and generating the first evaluation result based on the first evaluation data and the additional information. Therefore, in addition to the first evaluation data, the multifaceted first evaluation result considering the feature of the user can be generated. Accordingly, the evaluation to the biometric information of the user can be generated with higher accuracy.

### (Third Embodiment: Biometric Information Computing System 100)

Next, an example of a biometric information computing system 100 in the third embodiment will be described. The difference between the above-described embodiments and the third embodiment is that the first evaluation result is used in addition to the second evaluation data in generating the second evaluation result. For the contents similar to the above-described embodiments, the explanation will be omitted.

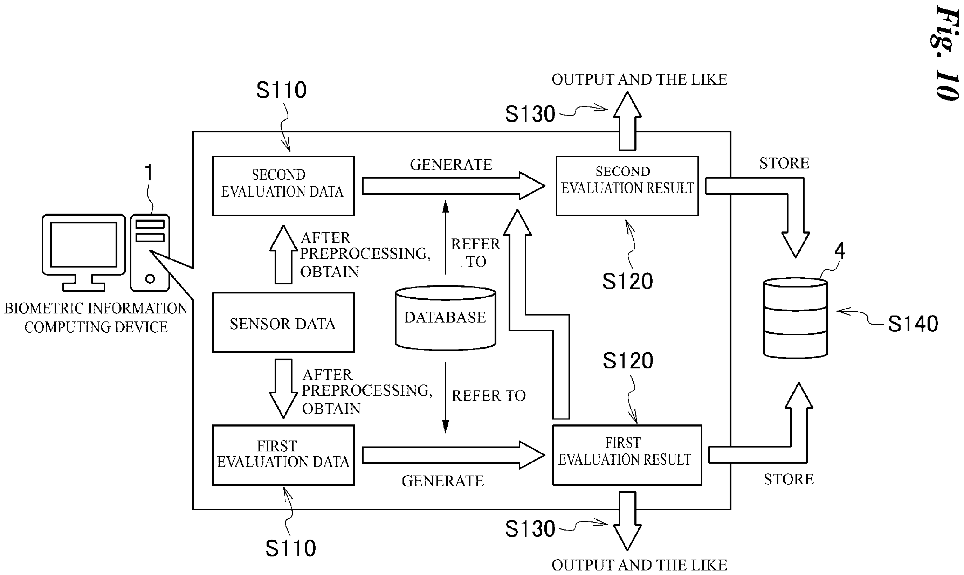

In the biometric information computing system 100 according to this embodiment, for example, as illustrated in Fig. 10, the generating step S120 includes referring to the database and generating the second evaluation result based on the first evaluation result and the second evaluation data. For example, the generating unit 12 generates the first evaluation result, and then generates the second evaluation result.

The generating unit 12 may determine the computing method to the second evaluation data, for example, corresponding to the contents of the first evaluation result. In this case, functions or the like different between the features of the contents of the first evaluation result are included in the classification information. The generating unit 12 may generate the second evaluation result, for example, based on the information in which the first evaluation result is combined with the second evaluation data.

According to this embodiment, in addition to the effects of the above-described embodiments, the generating unit 12 generates the second evaluation result based on the first evaluation result and the second evaluation data. Therefore, the second evaluation result based on the first evaluation result can be generated. Accordingly, the evaluation to the biometric information of the user can be generated with higher accuracy.

### (Fourth Embodiment: Biometric Information Computing System 100)

Next, an example of a biometric information computing system 100 in the fourth embodiment will be described. The difference between the above-described embodiments and the fourth embodiment is that attribute-based classification information appropriate for the evaluation data is selected from a plurality of pieces of the attribute-based classification information included in the classification information. For the contents similar to the above-described embodiments, the explanation will be omitted.

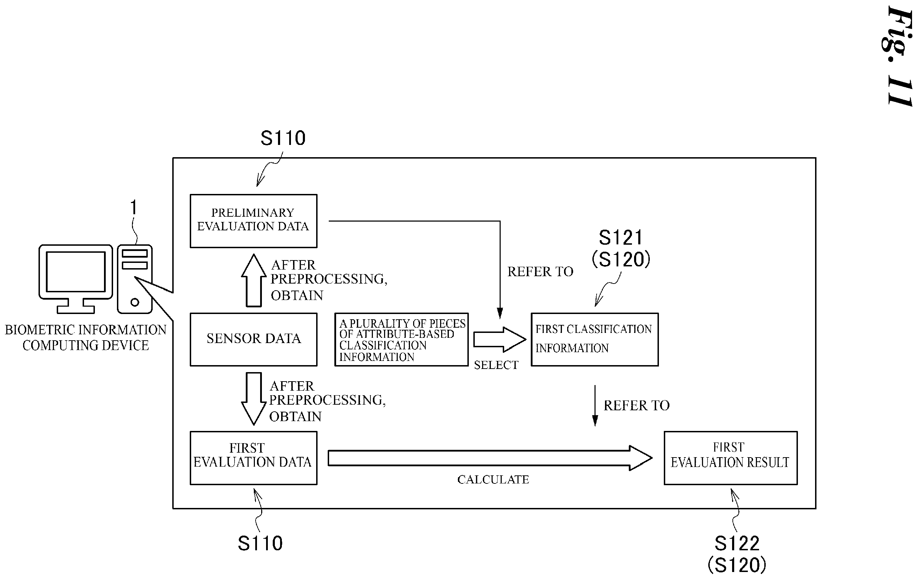

In a biometric information computing device 1 according to this embodiment, for example, as illustrated in Fig. 11, the generating step S 120 includes a selecting step S 121 and an attribute-based generating step S122. In Fig. 11, the description of the contents of the second evaluation data and the second evaluation result will be omitted.

The selecting step S121 refers to preliminary evaluation data, and selects specific attribute-based classification information (for example, first classification information) among a plurality of pieces of attribute-based classification information. The selecting step S 121 can be executed by, for example, a selecting unit included in the generating unit 12. The preliminary evaluation data indicates the feature different from the first evaluation data, and indicates, for example, the feature similar to the second evaluation data. For example, the second evaluation data may be used as the preliminary evaluation data.

The attribute-based generating step S122 refers to the selected first classification information, and calculates first biometric information (for example, value of blood carbon dioxide partial pressure) to the first evaluation data, thereby generating the first evaluation result. The attribute-based generating step S122 can be executed by, for example, an attribute-based generating unit included in the generating unit 12.

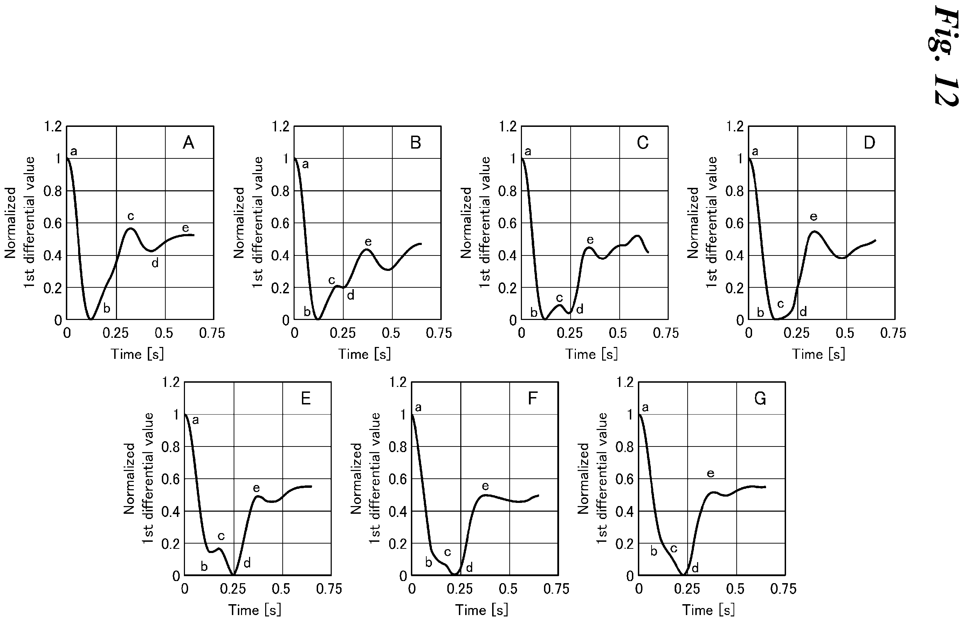

The plurality of pieces of attribute-based classification information are calculated using mutually different training data. For example, when data corresponding to the acceleration pulse wave of the examinee is used as the input data of the training data, for example, the input data is prepared for each of seven kinds (A to G) as illustrated in Fig. 12, thus generating seven kinds of attribute classification information.

When the plurality of pieces of attribute-based classification information are stored in the database, for example, the obtaining unit 11 obtains the evaluation data corresponding to the acceleration pulse wave of the user and the preliminary evaluation data. Then, the generating unit 12 refers to the preliminary evaluation data, and selects the first classification information. Subsequently, the generating unit 12 refers to the first classification information, and generates the first evaluation result for the first evaluation data. Therefore, among the pieces of the attribute classification information, the optimal classification information for the user can be selected.

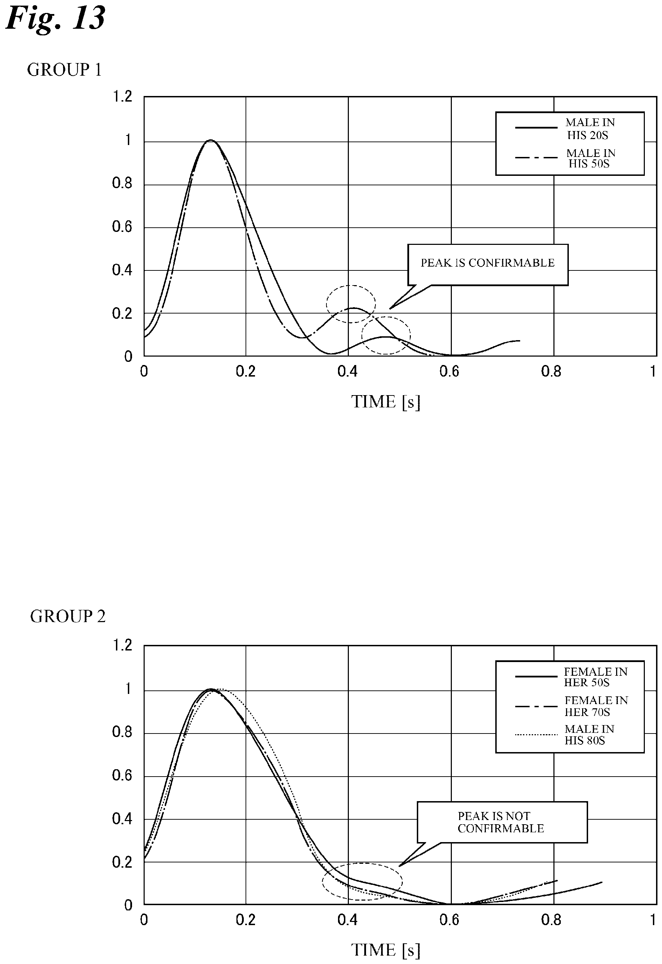

For example, when data corresponding to the velocity pulse wave of the examinee is used as the input data of the training data, for example, the input data may be prepared for each of two kinds (group 1 and group 2) as illustrated in Fig. 13, and two kinds of attribute classification information may be generated.

Here, while the data corresponding to the acceleration pulse wave illustrated in Fig. 12 is easily classified in detail based on the feature, in calculating the biometric information, the reduction in accuracy due to the false detection of the peak or the like is concerned. While the data corresponding to the velocity pulse wave illustrated in Fig. 13 has a difficulty in detailed classification based on the feature compared with the data corresponding to the acceleration pulse wave, the biometric information can be calculated with high accuracy because of the less false detection of the peak or the like.

Based on the above-described circumstances, the plurality of pieces of attribute classification information may include, for example, the data corresponding to the acceleration pulse wave as illustrated in Fig. 12 as selection data used for selecting the specific classification information, and for the training data in generating the attribute classification information, the data corresponding to the velocity pulse wave may be used.

In this case, as the obtaining step S 110, for example, the obtaining unit 11 obtains data corresponding to the velocity pulse wave from the sensor data based on the user's pulse wave as the first evaluation data. The obtaining unit 11 obtains data corresponding to the acceleration pulse wave from the sensor data as the preliminary evaluation data.

Next, as the selecting step S 121, for example, the generating unit 12 refers to the preliminary evaluation data, identifies selection data (first selection data) most similar to the preliminary evaluation data among a plurality of selection data including the data corresponding to the acceleration pulse wave, and selects first classification information associated with the first selection data. Then, as the attribute-based generating step S 122, the generating unit 12 refers to the first classification information, and generates the first evaluation result for the first evaluation data. This allows attempting the further improvement of the evaluation accuracy.

Here, an example of the data used for the above-described selection data or the like will be described.

For example, as illustrated in Fig. 12, the acceleration pulse wave includes inflection points of ***a*** to ***e***. For example, when the normalization is performed with a point ***a*** as the maximum peak in the acceleration pulse wave, the inflection points of a point ***b**,* a point ***c**,* a point ***d**,* and a point ***e*** in the order from the point ***a**,* the point ***a*** set to 1, and the point ***b*** or the point ***d*** as the minimum value set to 0, the acceleration pulse wave can be classified into seven patterns using a method of classifying with the values of the respective inflection points and a magnitude relationship of differences of the values. First, when the values of the inflection points meet ***b** < **d**,* the acceleration pulse wave is classified into a pattern A or B. In the case of ***b** < **d*** and further ***c*** ≥ 0.5, the acceleration pulse wave is classified into the pattern A, and otherwise the pattern B. Next, when the values of the inflection points meet ***b*** ≈ ***d**,* the acceleration pulse wave is classified into a pattern C or D. In the case of ***b*** ≈ ***d*** and further ***c*** ≈ 0, the acceleration pulse wave is classified into the pattern D, and otherwise the pattern C. Finally, in the case of ***b** > **d**,* the acceleration pulse wave can be classified into any of a pattern E, F, or G. The acceleration pulse wave is classified into the pattern E in the case of ***b** > **d*** and further ***b*** < ***c,*** the pattern F in the case of ***b*** ≈ ***c**,* and the pattern G in the case of ***b** > **c**.*

For example, the generating unit 12 determines, for example, which pattern of Fig. 12 the preliminary evaluation data corresponds to, and identifies the first selection data. For example, when the input inflection point ***b*** of the preliminary evaluation data is smaller than the inflection point ***d*** and further the inflection point ***c*** ≥ 0.5 is met, the pattern A is identified as the first selection data. Accordingly, the biometric information can be accurately calculated by referring to the classification information appropriate for the feature of the first evaluation data.

According to this embodiment, in addition to the effects of the above-described embodiments, the generating unit 12 includes the selecting unit that selects the first classification information by referring to the preliminary evaluation data, and the attribute-based generating unit that generates the first evaluation result for the first evaluation data by referring to the first classification information. Therefore, the optimal first classification information to the feature of the pulse wave is selected, and then, the first evaluation result for the first evaluation data can be generated. This allows attempting the further improvement of the evaluation accuracy.

According to this embodiment, the obtaining unit 11 obtains the data corresponding to the velocity pulse wave based on the pulse wave as the first evaluation data. The obtaining unit 11 obtains the data corresponding to the acceleration pulse wave based on the pulse wave as the preliminary evaluation data. Therefore, the attribute classification information can be selected using the acceleration pulse wave that facilitates the classification of the feature of the pulse wave compared with the velocity pulse wave. Additionally, the first evaluation result can be generated using the velocity pulse wave that facilitates the calculation of the biometric information compared with the acceleration pulse wave. This allows attempting the further improvement of the evaluation accuracy.

### (Fourth Embodiment: First Modification of Biometric Information Computing System 100)

Next, a first modification of the biometric information computing system 100 in the fourth embodiment will be described. The difference between the above-described example of the fourth embodiment and the first modification is that the classification information is selected using the evaluation result. For the contents similar to the above-described embodiments, the explanation will be omitted.

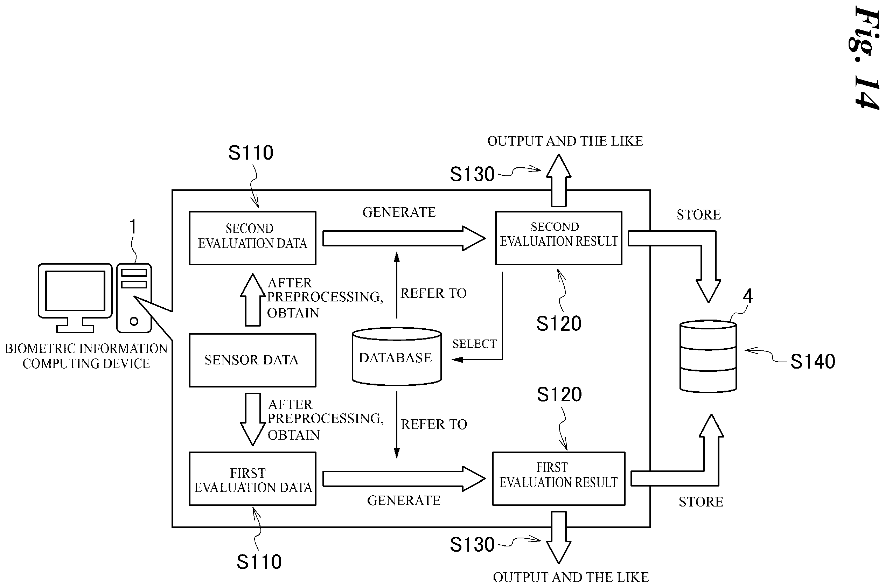

In this modification, for example, as illustrated in Fig. 14, the generating step S 120 selects first classification information among the classification information based on the second evaluation result, refers to the first classification information, and generates the first evaluation result for the first evaluation data. For example, similarly to the above-described selecting step S 121, the generating unit 12 refers to the second evaluation result, and selects specific attribute-based classification information among a plurality of pieces of the attribute-based classification information.

The generating unit 12 selects the first classification information based on, for example, the value of the biometric information included in the second evaluation result. In this respect, values for selection are preliminarily set to a plurality of pieces of attribute information.

Similarly to the above-described attribute-based generating step S 122, the generating unit 12 refers to the selected first classification information, and calculates the first biometric information for the first evaluation data, thus generating the first evaluation result.

According to this modification, the generating unit 12 selects the first classification information based on the second evaluation result, refers to the first classification information, and generates the first evaluation result for the first evaluation data. Therefore, the optimal first classification information is selected corresponding to the second evaluation result, and then, the first evaluation result for the first evaluation data can be generated. This allows attempting the further improvement of the evaluation accuracy.

### (Fourth Embodiment: Second Modification of Biometric Information Computing System 100)

Next, a second modification of the biometric information computing system 100 in the fourth embodiment will be described. The difference between the above-described example of the fourth embodiment and the second modification is that the first classification information is selected based on the feature of the pulse wave. For the contents similar to the above-described embodiments, the explanation will be omitted.

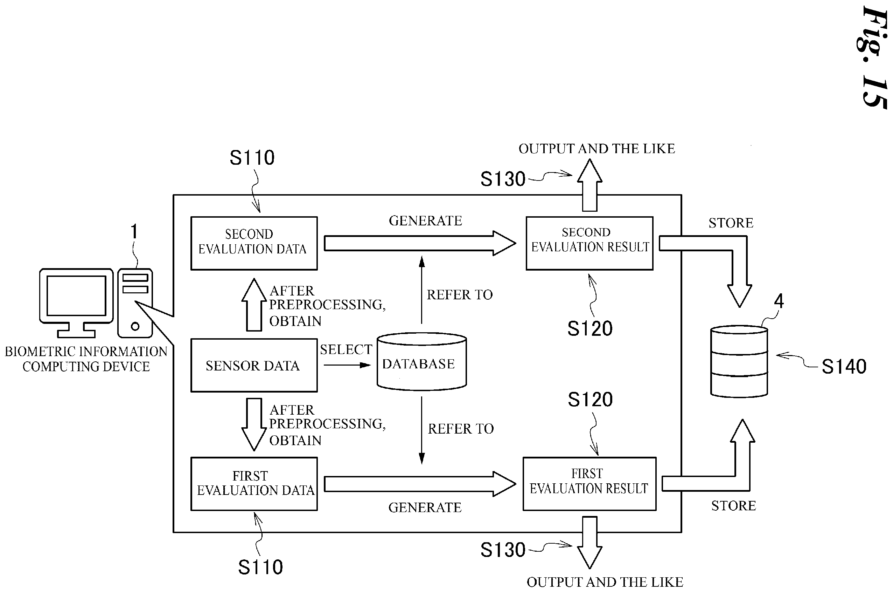

In this modification, for example, as illustrated in Fig. 15, the generating step S 120 selects first classification information based on the feature of the pulse wave (for example, sensor data) among the classification information, refers to the first classification information, and generates the first evaluation result for the first evaluation data. For example, similarly to the above-described selecting step S 121, the generating unit 12 refers to the sensor data, and selects specific attribute-based classification information among a plurality of pieces of the attribute-based classification information.

As "the feature of the pulse wave," for example, data after performing at least a part of the processes as illustrated in Fig. 4A to Fig. 4D to the sensor data may be used. Especially, using the pulse wave data after performing the filter process on the sensor data as the feature of the pulse wave allows attempting the accuracy improvement in selecting the specific attribute classification information.

The generating unit 12 selects the first classification information, for example, by comparing the above-described selection data with the feature of the pulse wave. The generating unit 12 may select the first classification information, for example, based on a half-value width or a relative intensity of the peak included in the sensor data or the like. In this respect, values for selection are preliminarily set to a plurality of pieces of attribute information.

Similarly to the above-described attribute-based generating step S 122, the generating unit 12 refers to the selected first classification information, and calculates the first biometric information for the first evaluation data, thus generating the first evaluation result.

According to this modification, the generating unit 12 selects the first classification information based on the feature of the pulse wave, refers to the first classification information, and generates the first evaluation result for the first evaluation data. Therefore, the optimal first classification information is selected corresponding to the feature of the pulse wave, and then, the first evaluation result for the first evaluation data can be generated. This allows attempting the further improvement of the evaluation accuracy.

### (Fourth Embodiment: Third Modification of Biometric Information Computing System 100)

Next, a third modification of the biometric information computing system 100 in the fourth embodiment will be described. The difference between the above-described example of the fourth embodiment and the third modification is that the first classification information is selected based on the additional information. For the contents similar to the above-described embodiments, the explanation will be omitted.

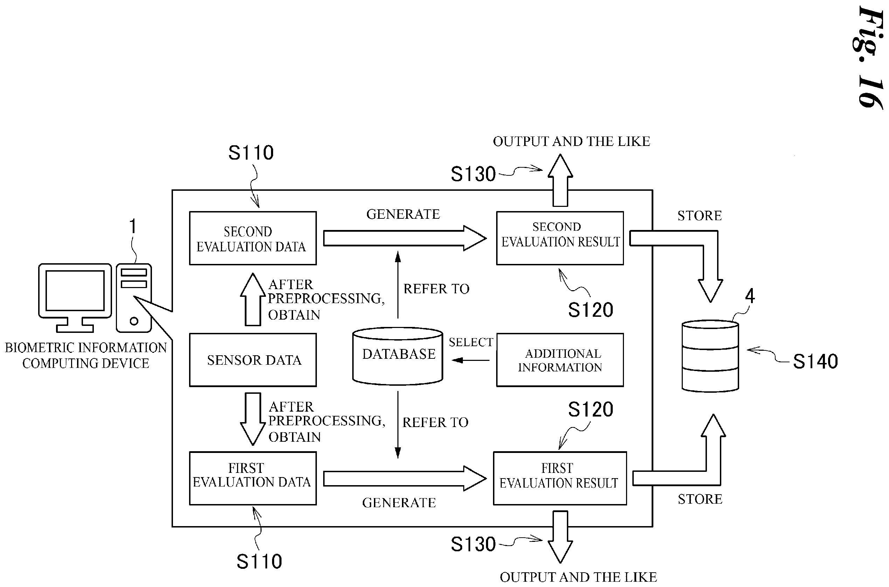

In this modification, for example, as illustrated in Fig. 16, the generating step S 120 selects first classification information based on the additional information among the classification information, refers to the first classification information, and generates the first evaluation result for the first evaluation data. For example, similarly to the above-described selecting step S 121, the generating unit 12 refers to the additional information, and selects specific attribute-based classification information among a plurality of pieces of the attribute-based classification information. The additional information is similar to the above-described additional information.

The generating unit 12 may select the first classification information, for example, based on attribute information or the like such as an age and a gender included in the additional information. In this respect, attribute information or the like for selection is preliminarily set to a plurality of pieces of attribute information.

Similarly to the above-described attribute-based generating step S 122, the generating unit 12 refers to the selected first classification information, and calculates the first biometric information for the first evaluation data, thus generating the first evaluation result.

According to this modification, the generating unit 12 selects the first classification information based on the additional information, refers to the first classification information, and generates the first evaluation result for the first evaluation data. Therefore, the optimal first classification information is selected corresponding to the feature of the additional information, and then, the first evaluation result for the first evaluation data can be generated. This allows attempting the further improvement of the evaluation accuracy.

### (Fifth Embodiment: Biometric Information Computing System 100)

Next, an example of a biometric information computing system 100 in the fifth embodiment will be described. The difference between the above-described embodiments and the fifth embodiment is that a calculating step S 160 is included. For the contents similar to the above-described embodiments, the explanation will be omitted.

The biometric information computing system 100 according to this embodiment includes, for example, a calculating step S 160. In the biometric information computing system 100, for example, the calculating step S160 is performed after the above-described storing step S140.

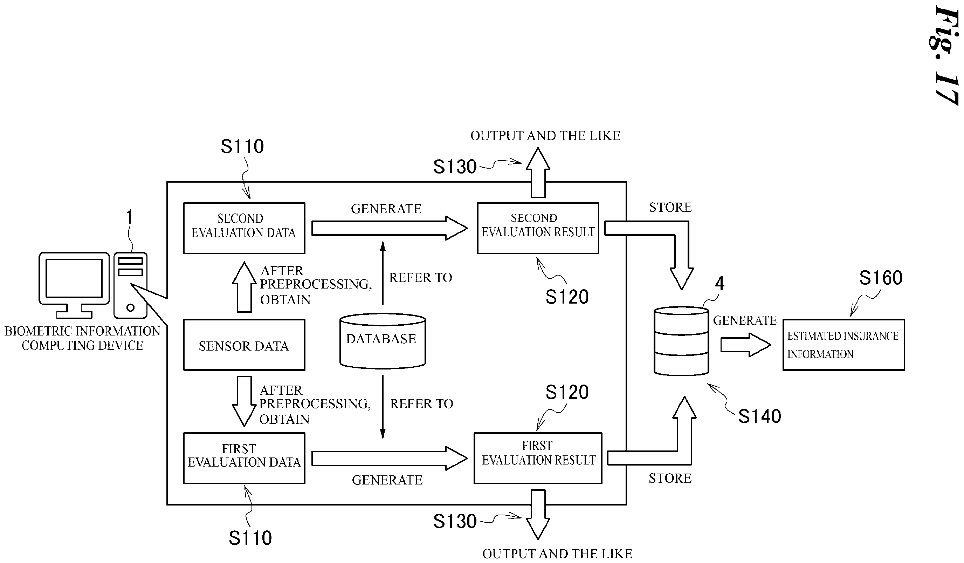

For example, as illustrated in Fig. 17, the calculating step S160 generates a comprehensive evaluation result comprehensively evaluating the feature of the user based on a plurality of evaluation results (for example, first evaluation result and second evaluation result) stored in the server 4 or the storage unit 104. The calculating step S160 can be executed by, for example, a comprehensive evaluation unit included in the generating unit 12 of the biometric information computing device 1, and additionally, may be executed by, for example, a comprehensive evaluation unit included in the server 4.

The comprehensive evaluation result is similar to that in the above-described embodiment, and the following describes estimated insurance information including an insurance premium as an example.

The comprehensive evaluation unit may generate the estimated insurance information by referring to, for example, the data format that is preliminarily stored in the storage unit 104 or the like and recognizable by the user. The comprehensive evaluation unit may generate the appropriate estimated insurance information to the plurality of evaluation results by referring to, for example, a database.

In the database, for example, similarly to the above-described database, insurance classification information for generating the estimated insurance information to the plurality of evaluation results may be stored. In the database, one or more pieces of the insurance classification information are stored, and additionally, for example, a plurality of insurance training data used for generating the insurance classification information may be stored.

The insurance classification information is, for example, a function indicating a correlation between a plurality of past evaluation results (insurance input data) which is preliminarily obtained and insurance reference data associated with the insurance input data. The insurance reference data includes an insurance premium to insurance input data proven in the past. The insurance classification information is generated using a plurality of insurance training data each of which is a pair of the insurance input data and the insurance reference data.

The insurance classification information indicates a calibration model generated based on a result of an analysis performed by, for example, the above-described regression analysis having the insurance input data as an explanatory variable and the insurance reference data as an objective variable. For the insurance classification information, for example, the calibration model (insurance calibration model) can be regularly updated. The insurance classification information may include, similarly to the above-described classification information, for example, a trained model (insurance trained model) generated by machine learning using a plurality of insurance training data.

According to this embodiment, in addition to the effects of the above-described embodiments, the comprehensive evaluation unit generates the comprehensive evaluation result based on the first evaluation result and the second evaluation result. Therefore, for each of the evaluation results, the evaluation considering the feature of the user can be achieved. Accordingly, the evaluation result appropriate for each user can be generated. Especially, when the estimated insurance information is used as the comprehensive evaluation result, the variation of estimated insurance premium or the like due to a subjective view of the user or the like can be suppressed.

### (Sixth Embodiment: Biometric Information Computing System 100)

Next, an example of a biometric information computing system 100 in the sixth embodiment will be described. The difference between the above-described embodiments and the sixth embodiment is that a determination result is used. For the contents similar to the above-described embodiments, the explanation will be omitted.

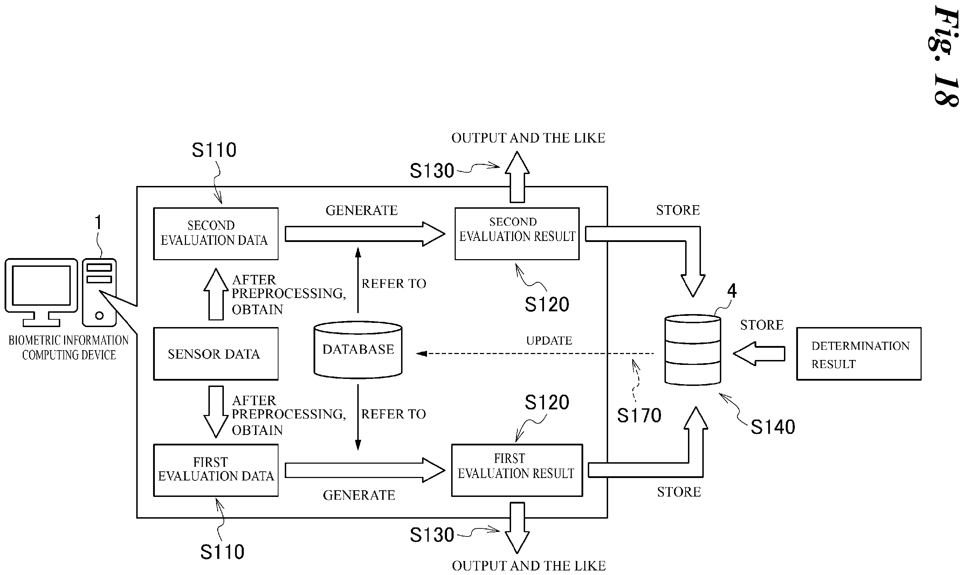

In the biometric information computing system 100 according to this embodiment, for example, as illustrated in Fig. 18, the storing step S 140 obtains determination results determined to the contents of the plurality of evaluation results (for example, first evaluation result and second evaluation result) by the user, and stores the determination results and the plurality of evaluation results in the manner of being mutually associated. The storing step S 140 can be executed by, for example, the storing unit 14 or the server 4.

The determination result can be obtained by, for example, inputting the result of comparison between the plurality of output evaluation results and the biometric information measured using a publicly known measuring device via the input unit 108 or the like by the user.

The biometric information computing system 100 according to this embodiment may include, for example, an updating step S 170. In this case, the updating step S170 can be executed by, for example, a learning unit 15, and may be executed by, for example, the server 4.

The learning unit 15 updates the classification information based on the determination result and the plurality of evaluation results stored in the storing step S140. The learning unit 15 updates the classification information using, for example, a publicly known technique.

According to this embodiment, in addition to the effects of the above-described embodiments, the storing unit 14 or the server 4 stores the determination result, the first evaluation result, and the second evaluation result in the manner of being mutually associated. Therefore, the evaluation results can be each easily compared with the determination result.

According to this embodiment, the learning unit 15 updates the classification information based on the determination result, the first evaluation result, and the second evaluation result. Therefore, when the accuracy of each evaluation result is decreased, the accuracy can be easily improved. Accordingly, the improvement of accuracy in the evaluation of the biometric information can be maintained.

### (Seventh Embodiment: Biometric Information Computing System 100)

Next, an example of a biometric information computing system 100 in the seventh embodiment will be described. The difference between the above-described embodiments and the seventh embodiment is that an electronic device 2 is used. For the contents similar to the above-described embodiments, the explanation will be omitted.

In the biometric information computing system 100 according to this embodiment, in addition to that the electronic device 2 is used instead of the above-described biometric information computing device 1, for example, each of the biometric information computing device 1 and the electronic device 2 may be used corresponding to the situation.

Similarly to the biometric information computing device 1, the electronic device 2 indicates an electronic device such as a personal computer as described above. The configuration of the electronic device 2 may include, for example, a configuration similar to that of Fig. 5A. The respective functions for achieving the biometric information computing program of the electronic device 2 are achieved by, similarly to the biometric information computing device 1, executing programs stored in the storage unit 104 or the like by the CPU 101 with the RAM 103 as the work area.

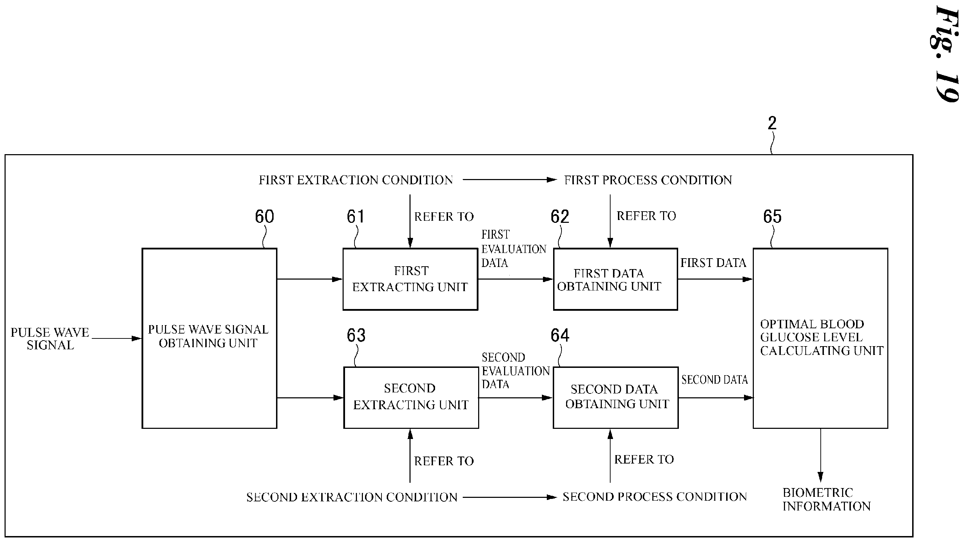

Fig. 19 illustrates a sequence for achieving a biometric information computing program of the electronic device 2. The electronic device 2 includes a pulse wave signal obtaining unit 60, a first extracting unit 61 and a second extracting unit 63 connected to the pulse wave signal obtaining unit 60, a first data obtaining unit 62 connected to the first extracting unit 61, a second data obtaining unit 64 connected to the second extracting unit 63, and an optimal blood glucose level calculating unit 65 connected to the first data obtaining unit 62 and the second data obtaining unit 64.

The pulse wave signal obtaining unit 60 obtains a pulse wave signal transmitted from the sensor 5, the server 4, and another electronic device via the communications network 3. The pulse wave signal obtaining unit 60 outputs the obtained pulse wave signal to the first extracting unit 61 and the second extracting unit 63. The pulse wave signal has a feature similar to that of the above-described sensor data.

The first extracting unit 61 refers to a first extraction condition, and extracts first evaluation data based on the pulse wave signal input from the pulse wave signal obtaining unit 60. The first extracting unit 61 outputs the extracted first evaluation data to the first data obtaining unit 62.

The first data obtaining unit 62 refers to a first process condition associated with the first extraction condition, processes the first evaluation data input from the first extracting unit 61, thus obtaining first data. The first data obtaining unit 62 outputs the obtained first data to the optimal blood glucose level calculating unit 65.

The second extracting unit 63 refers to a second extraction condition, and extracts second evaluation data based on the pulse wave signal input from the pulse wave signal obtaining unit 60. The second extracting unit 63 outputs the extracted second evaluation data to the second data obtaining unit 64.

The second data obtaining unit 64 refers to a second process condition associated with the second extraction condition, processes the second evaluation data input from the second extracting unit 63, thus obtaining second data. The second data obtaining unit 64 outputs the obtained second data to the optimal blood glucose level calculating unit 65.

The optimal blood glucose level calculating unit 65 calculates biometric information as an optimal value based on the first data input from the first data obtaining unit 62 and the second data input from the second data obtaining unit 64.

The optimal blood glucose level calculating unit 65 is not necessarily included in the electronic device 2, and the optimal blood glucose level calculating unit 65 may output the first data and the second data as the biometric information.

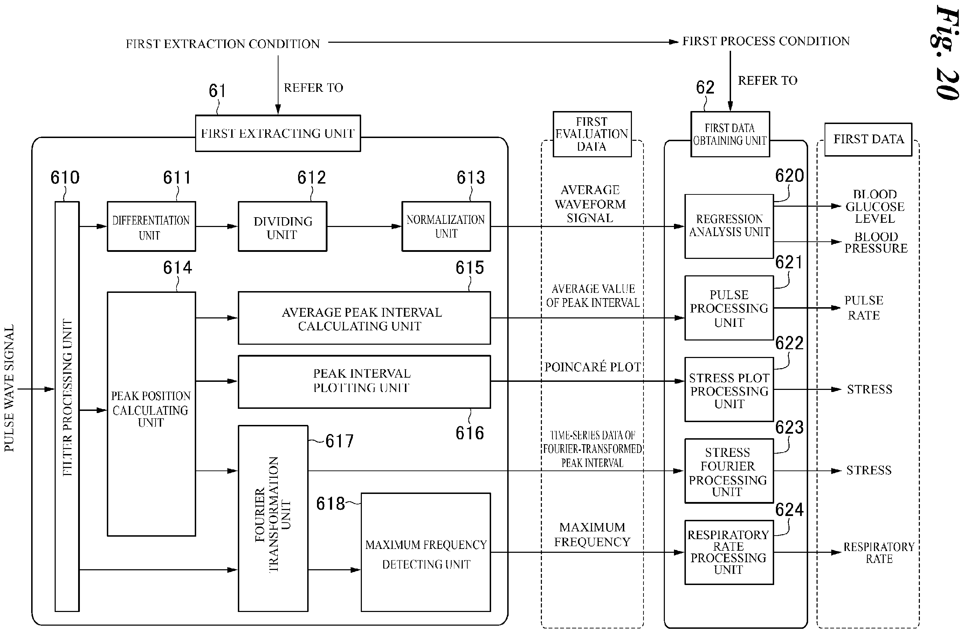

Fig. 20 illustrates an example of a specific configuration of the first extracting unit 61 and the first data obtaining unit 62. The first extracting unit 61 includes a filter processing unit 610, a differentiation unit 611 connected to the filter processing unit 610, a peak position calculating unit 614 connected to the filter processing unit 610, a dividing unit 612 connected to the differentiation unit 611, a normalization unit 613 connected to the dividing unit 612, an average peak interval calculating unit 615 connected to the peak position calculating unit 614, a peak interval plotting unit 616 connected to the peak position calculating unit 614, a Fourier transformation unit 617 connected to the peak position calculating unit 614, and a maximum frequency detecting unit 618 connected to the Fourier transformation unit 617.

The filter processing unit 610 performs a filtering process on the obtained pulse wave signal. In the filter processing unit 610, a bandpass filter of, for example, 0.5 to 5 Hz is used for filtering, but it is not limited to this. The filter processing unit 610 refers to the first extraction condition, and determines an extraction method for extracting the first evaluation data from the obtained pulse wave signal. The filter processing unit 610 outputs the filter-processed pulse wave signal to at least any one of the differentiation unit 611, the peak position calculating unit 614, and the Fourier transformation unit 617 based on the determined extraction method.

The first extracting unit 61 does not necessarily use all of the extraction methods included in the first extracting unit 61 for obtaining one first evaluation data, but extracts the first evaluation data from the pulse wave signal with at least one extraction method determined by the filter processing unit 610.

The differentiation unit 611 differentiates the pulse wave signal input from the filter processing unit 610. When the filter processing unit 610 determines the differential process on be necessary, the differentiation unit 611 performs a differential process on the input pulse wave signal. The differentiation unit 611 outputs the processed pulse wave signal to the dividing unit 612.

The dividing unit 612 divides each of a plurality of waveform signals input from the differentiation unit 611 into divided waveform data of integer cycles. In the dividing unit 612, while the integer cycle is set to one cycle in this embodiment, the integer cycle may be multiple cycles. The dividing unit 612 outputs the divided waveform data to the normalization unit 613.

The normalization unit 613 performs normalization for making time widths of a plurality of divided waveform signals input from the dividing unit 612 uniform, obtains an average waveform signal that is an average of the plurality of divided waveform signals, and performs normalization with a maximum value of 1 and a minimum value of 0 of an amplitude of the average waveform signal. The normalization unit 613 outputs the normalized average waveform signal to a regression analysis unit 620.

The normalization unit 613 may perform trimming of the plurality of divided waveform signals input from the dividing unit 612 with a certain time width or a certain sampling number for making the time widths of the divided waveform signals uniform. The processing method for making the time width uniform by the normalization unit 613 is determined by the filter processing unit 610.

While the normalization unit 613 requires a plurality of divided waveform signals in obtaining the average waveform signal, the number of the required divided waveform signals is determined by the filter processing unit 610.

The peak position calculating unit 614 calculates a peak position of the pulse wave signal input from the filter processing unit 610 and a peak interval that is a distance between the adjacent peaks. The peak position calculating unit 614 outputs the calculated peak interval to at least any one of the average peak interval calculating unit 615, the peak interval plotting unit 616, and the Fourier transformation unit 617 based on the extraction method.

The average peak interval calculating unit 615 calculates the average of the peak intervals input from the peak position calculating unit 614, divides the average value of the peak interval by a sampling rate of a measuring device, and converts it into the number of seconds. The average peak interval calculating unit 615 outputs the average value converted into the number of seconds to a pulse processing unit 621.

The peak interval plotting unit 616 plots a graph having the horizontal axis indicating the peak interval input from the peak position calculating unit 614 and the vertical axis indicating the peak interval next to the above-described peak interval, thereby obtaining Poincaré plot of the peak interval of the pulse wave. The peak interval plotting unit 616 outputs the Poincaré plot of the peak interval of the pulse wave to a stress plot processing unit 622.

When the peak interval is input from the peak position calculating unit 614, the Fourier transformation unit 617 performs Fourier transformation of data converted from the peak interval into time-series data. The Fourier transformation unit 617 may perform Fourier transformation with the pulse wave signal processed by the filter processing unit 610 as the input. The Fourier transformation unit 617 outputs the Fourier-transformed signal to at least any of the maximum frequency detecting unit 618 and a stress Fourier processing unit 623 based on the extraction method.

The maximum frequency detecting unit 618 detects a maximum frequency, which is a frequency having a maximum value in a range from 0.15 to 0.35 Hz, of the signal input from the Fourier transformation unit 617. The maximum frequency detecting unit 618 outputs the detected maximum frequency to a respiratory rate processing unit 624.

The first data obtaining unit 62 includes the regression analysis unit 620 connected to the normalization unit 613, the pulse processing unit 621 connected to the average peak interval calculating unit 615, the stress plot processing unit 622 connected to the peak interval plotting unit 616, the stress Fourier processing unit 623 connected to the Fourier transformation unit 617, and the respiratory rate processing unit 624 connected to the maximum frequency detecting unit 618.

For example, as a method of processing, the first data obtaining unit 62, obtains the first data from the first evaluation data using a calibration model established based on a correlation between a measured value of biometric information and a preliminarily obtained pulse wave signal.

The regression analysis unit 620 obtains, for example, the blood glucose level, the blood pressure, the blood oxygen saturation, the blood carbon dioxide concentration, or the like as the first data from the normalized average waveform signal input from the normalization unit 613 based on the calibration model.

The regression analysis unit 620 can obtain the first data using a preliminarily established calibration model for general use by storing it in the first data obtaining unit 62.

The pulse processing unit 621 divides the average value of the peak interval input from the average peak interval calculating unit 615 by the sampling rate, thereby converting the average value of the peak interval into the number of seconds. The pulse processing unit 621 divides 60 seconds by the calculated number of seconds to calculate the pulse rate (bpm) per minute, thus obtaining the pulse rate as the first data.

The stress plot processing unit 622 obtains, for example, the stress level as the first data from the Poincaré plot input from the peak interval plotting unit 616. The stress plot processing unit 622 calculates a variance value of the Poincaré plot, and estimates the stress level from the magnitude of the variance value.

The stress Fourier processing unit 623 obtains, for example, the stress level as the first data from an integral ratio of the signal input from the Fourier transformation unit 617. As a specific method, for example, the stress Fourier processing unit 623 calculates a power spectral density (PSD) from the Fourier-transformed time-series data of the peak interval using an autoregressive model, and determines the stress level by a ratio between integral values of respective sums of intensities of a low frequency LF (Low Frequency) as a region from 0.5 Hz to 0.15 Hz and a high frequency HF (Hi Frequency) as a region from 0.15 Hz to 0.40 Hz of a power spectrum.

The respiratory rate processing unit 624 multiplies the frequency having the maximum value input from the maximum frequency detecting unit 618 by 60 seconds to convert the frequency into the respiratory rate (bpm) per minute, thus obtaining the respiratory rate as the first data.

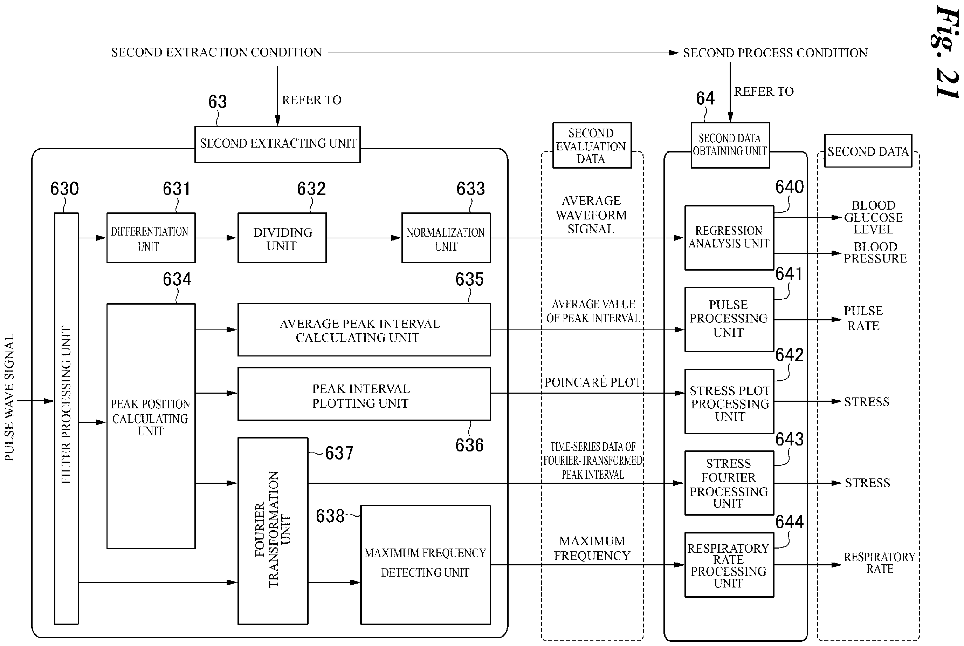

Fig. 21 illustrates an example of a specific configuration of the second extracting unit 63 and the second data obtaining unit 64. The second extracting unit 63 includes a filter processing unit 630, a differentiation unit 631 connected to the filter processing unit 630, a peak position calculating unit 634 connected to the filter processing unit 630, a dividing unit 632 connected to the differentiation unit 631, a normalization unit 633 connected to the dividing unit 632, an average peak interval calculating unit 635 connected to the peak position calculating unit 634, a peak interval plotting unit 636 connected to the peak position calculating unit 634, a Fourier transformation unit 637 connected to the peak position calculating unit 634, and a maximum frequency detecting unit 638 connected to the Fourier transformation unit 637. The second extracting unit 63 is configured with the same one as the first extracting unit 61, and while the first extracting unit 61 refers to the first extraction condition, the second extracting unit 63 refers to the second extraction condition instead of the first extraction condition.

The filter processing unit 630 performs a filtering process on the obtained pulse wave signal. In the filter processing unit 630, a bandpass filter of, for example, 0.5 to 5 Hz is used for filtering, but it is not limited to this. The filter processing unit 630 refers to the second extraction condition, and determines an extraction method for extracting the first evaluation data from the obtained pulse wave signal. The filter processing unit 630 outputs the filter-processed pulse wave signal to at least any one of the differentiation unit 631, the peak position calculating unit 634, and the Fourier transformation unit 637 based on the determined extraction method.

The second extracting unit 63 does not necessarily use all of the extraction methods included in the second extracting unit 63 for obtaining one second evaluation data, but extracts the second evaluation data from the pulse wave signal with at least one extraction method determined by the filter processing unit 630.

The differentiation unit 631 differentiates the pulse wave signal input from the filter processing unit 630. When the filter processing unit 630 determines the differential process on be necessary, the differentiation unit 631 performs a differential process on the input pulse wave signal. The differentiation unit 631 outputs the differentiated pulse wave signal to the dividing unit 632.

The dividing unit 632 divides each of a plurality of waveform signals input from the differentiation unit 631 into divided waveform data of integer cycles. In the dividing unit 632, while the integer cycle is set to one cycle in this embodiment, the integer cycle may be multiple cycles. The dividing unit 632 outputs the divided waveform data to the normalization unit 633.

The normalization unit 633 performs normalization for making time widths of a plurality of divided waveform signals input from the dividing unit 632 uniform, obtains an average waveform signal that is an average of the plurality of divided waveform signals, and performs normalization with a maximum value of 1 and a minimum value of 0 of an amplitude of the average waveform signal. The normalization unit 633 outputs the normalized average waveform signal to a regression analysis unit 640.

The normalization unit 633 may perform trimming of the plurality of divided waveform signals input from the dividing unit 632 with a certain time width or a certain sampling number for making the time widths of the divided waveform signals uniform. The processing method for making the time width uniform by the normalization unit 633 is determined by the filter processing unit 630.

While the normalization unit 633 requires a plurality of divided waveform signals in obtaining the average waveform signal, the number of the required divided waveform signals is determined by the filter processing unit 630.

The peak position calculating unit 634 calculates a peak position of the pulse wave signal input from the filter processing unit 630 and a peak interval that is a distance between the adjacent peaks. The peak position calculating unit 634 outputs the calculated peak interval to at least any one of the average peak interval calculating unit 635, the peak interval plotting unit 636, and the Fourier transformation unit 637 based on the extraction method.

The average peak interval calculating unit 635 calculates the average of the peak intervals input from the peak position calculating unit 634, divides the average value of the peak interval by a sampling rate of a measuring device, and converts it into the number of seconds. The average peak interval calculating unit 635 outputs the average value converted into the number of seconds to a pulse processing unit 641.

The peak interval plotting unit 636 plots a graph having the horizontal axis indicating the peak interval input from the peak position calculating unit 634 and the vertical axis indicating the peak interval next to the above-described peak interval, thereby obtaining Poincaré plot of the peak interval of the pulse wave. The peak interval plotting unit 636 outputs the Poincaré plot of the peak interval of the pulse wave to a stress plot processing unit 642.

When the peak interval is input from the peak position calculating unit 634, the Fourier transformation unit 637 performs Fourier transformation of data converted from the peak interval into time-series data. The Fourier transformation unit 637 may perform Fourier transformation with the pulse wave signal processed by the filter processing unit 630 as the input. The Fourier transformation unit 637 outputs the Fourier-transformed signal to at least any one of the maximum frequency detecting unit 638 and a stress Fourier processing unit 643 based on the extraction method.

The maximum frequency detecting unit 638 detects a maximum frequency, which is a frequency having a maximum value in a range from 0.15 to 0.35 Hz, of the signal input from the Fourier transformation unit 637. The maximum frequency detecting unit 638 outputs the detected maximum frequency to a respiratory rate processing unit 644.

The second data obtaining unit 64 includes the regression analysis unit 640 connected to the normalization unit 633, the pulse processing unit 641 connected to the average peak interval calculating unit 635, the stress plot processing unit 642 that obtains the stress level from the graph plotted by the peak interval plotting unit 636, the stress Fourier processing unit 643 connected to the Fourier transformation unit 637, and the respiratory rate processing unit 644 connected to the maximum frequency detecting unit 638.

The second data obtaining unit 64 refers to the second process condition associated with the second extraction condition and processes the second evaluation data extracted by the second extracting unit 63, thus obtaining the second data.

For example, as a method of processing, the second data obtaining unit 64 obtains the second data from the second evaluation data using a calibration model established based on a correlation between a measured value of biometric information and a preliminarily obtained pulse wave signal.

The regression analysis unit 640 obtains, for example, the blood glucose level, the blood pressure, the blood oxygen saturation, the blood carbon dioxide concentration, or the like as the second data from the normalized average waveform signal input from the normalization unit 633 based on the calibration model.

The regression analysis unit 640 can obtain the second data using a preliminarily established calibration model for general use as general-purpose data by storing it in the second data obtaining unit 64.

The pulse processing unit 641 divides the average value of the peak interval input from the average peak interval calculating unit 635 by the sampling rate, thereby converting the average value of the peak interval into the number of seconds. The pulse processing unit 641 divides 60 seconds by the calculated number of seconds to calculate the pulse rate (bpm) per minute, thus obtaining the pulse rate as the second data.

The stress plot processing unit 642 obtains, for example, the stress level as the second data from the Poincaré plot input from the peak interval plotting unit 636. The stress plot processing unit 642 calculates a variance value of the Poincaré plot, and estimates the stress level from the magnitude of the variance value.

The stress Fourier processing unit 643 obtains, for example, the stress level as the second data from an integral ratio of the signal input from the Fourier transformation unit 637.

The respiratory rate processing unit 644 multiplies the frequency having the maximum value input from the maximum frequency detecting unit 638 by 60 seconds to convert the frequency into the respiratory rate (bpm) per minute, thus obtaining the respiratory rate as the second data.

The first data and the second data obtained by the biometric information computing system 100 include, for example, at least one of the blood pressure, the blood glucose level, the blood oxygen saturation, the blood carbon dioxide concentration, the pulse rate, the respiratory rate, the stress level, the vascular age, the degree of diabetes, and the like.

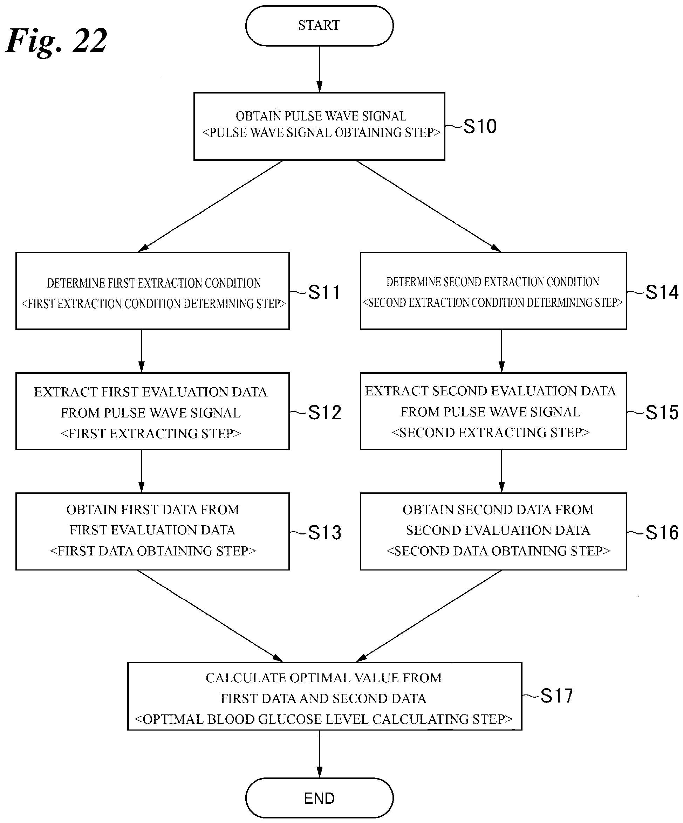

Next, an example of the operation of the biometric information computing system 100 according to this embodiment will be described. Fig. 22 is a flowchart illustrating an example of the operation of the biometric information computing system 100 according to this embodiment.

In a pulse wave signal obtaining step S10, the obtaining unit 50 obtains the pulse wave signal, and outputs the pulse wave signal to the communication I/F 51 via the internal bus 54. At this time, instead of the pulse wave signal obtained by the obtaining unit 50, the biometric information computing system 100 may output the pulse wave signal stored in the memory 52 to the communication I/F 51 via the internal bus 54. As a specific method for obtaining the pulse wave signal, for example, an FBG sensor is used.

Next, the communication I/F 51 to which the pulse wave signal has been input from the obtaining unit 50 transmits the pulse wave signal to the pulse wave signal obtaining unit 60 via the communications network 3. At this time, instead of the pulse wave signal obtained by the obtaining unit 50, the pulse wave signal stored in the server 4 may be transmitted to the pulse wave signal obtaining unit 60.

Next, the pulse wave signal obtaining unit 60 to which the pulse wave signal has been transmitted via the communications network 3 outputs the pulse wave signal to the first extracting unit 61 and the second extracting unit 63.

Next, in a first extraction condition determining step S11, the first extracting unit 61 inputs the pulse wave signal input from the pulse wave signal obtaining unit 60 to the filter processing unit 610. Then, the filter processing unit 610 determines the extraction method performed to the input pulse wave signal by referring to the first extraction condition.

The filter processing unit 610 determines the extraction method performed to the pulse wave signal by the first extracting unit 61 from the first extraction conditions depending on the state of the obtained pulse wave signal, the biometric information desired to be obtained, and an external factor. The external factor includes, for example, at least one of the user's information such as an age, a gender, a case history, lifestyle habits, medication information, a degree of arteriosclerosis, a health condition, and genetic information of the user, and environment information such as a temperature and a humidity. For example, when, as the biometric information desired to be obtained, the blood glucose level is desired to be obtained, the filter processing unit 610 instructs the first extracting unit 61 to output the pulse wave signal to the differentiation unit 611 after the pulse wave signal is processed by the filter processing unit 610. The first extraction condition is a data group including a list of extraction methods for extracting the first evaluation data from the pulse wave signal. The data group of the first extraction condition may include a plurality of extraction methods, and may include a predetermined single extraction method.

Next, in the first extracting step S12, the first extracting unit 61 extracts first evaluation data from the pulse wave signal input from the pulse wave signal obtaining unit 60. The first evaluation data is waveform data extracted from the pulse wave signal by the first extracting unit 61 for obtaining the biometric information by the first data obtaining unit 62. The first evaluation data is waveform data, for example, formed by processing the pulse wave signal into a waveform of one cycle or the like by at least any of a filtering process, a differential process, a normalization process, and an averaging process. In this embodiment, a biometric information computing system 100 for obtaining the blood glucose level will be described as an example.

First, the filter processing unit 610 performs the filtering process on the pulse wave signal input from the pulse wave signal obtaining unit 60, and then, for example, when the blood glucose level is obtained as the biometric information, based on the extraction method determined in the first extraction condition determining step S11, the filter processing unit 610 outputs the pulse wave signal to the differentiation unit 611.

Next, the differentiation unit 611 determines whether to differentiate the pulse wave signal input from the filter processing unit 610 or not based on the extraction method determined in the first extraction condition determining step S11, and after performing the process, outputs the pulse wave signal to the dividing unit 612.

The reason for determining whether to differentiate the pulse wave signal by the differentiation unit 611 or not based on the extraction method determined in the first extraction condition determining step S11 is to obtain the appropriate first evaluation data depending on the first data desired to be obtained because the feature of the obtained first evaluation data differs depending on whether to differentiate the pulse wave signal or not. "To differentiate the pulse wave signal" means extracting the pulse wave signal as an acceleration pulse wave, and "not to differentiate the pulse wave signal" means extracting the pulse wave signal as a velocity pulse wave.

Next, the dividing unit 612 divides each of a plurality of waveform signals input from the differentiation unit 611 into divided waveform data of one cycle to perform averaging. Then, the dividing unit 612 outputs the divided waveform data to the normalization unit 613.

The normalization unit 613 performs normalization of the horizontal axis for making time widths of a plurality of divided waveform signals input from the dividing unit 612 uniform, obtains an average waveform signal that is an average of the plurality of divided waveform signals, and performs normalization of the vertical axis with a maximum value of 1 and a minimum value of 0 of the average waveform signal. At this time, the first extracting unit 61 obtains the average waveform signal as the first evaluation data. Then, the normalization unit 613 outputs the normalized average waveform signal to the first data obtaining unit 62.

The reason for performing the normalization process of the horizontal axis for making the time width uniform by the normalization unit 613 is that since a difference is significantly generated in a terminating end side of the pulse wave, by deleting this part, the main body part of the pulse wave is obtained as the analysis object. The reason for performing the normalization process of the vertical axis with the maximum value of 1 and the minimum value of 0 of the average waveform signal is to average a variation of a pressing pressure in attaching the FBG sensor to the measurement position or a variation of measurement data due to the position shift of the FBG sensor in the measurement, and is to improve the accuracy of the correlation between the pulse wave signal and the measured value of the biometric information by reducing the noise caused by the variation in the measurement.

Next, the biometric information computing system 100 transitions to a first data obtaining step S13, refers to the first process condition associated with the first extraction condition, processes the first evaluation data input from the first extracting unit 61 by the first data obtaining unit 62, thus obtaining the first data.

The first process condition is a data group including the method associated with the first extraction condition for the process performed by the first data obtaining unit 62 to the first evaluation data input from the first extracting unit 61. The first data obtaining unit 62 determines the processing method from the first process condition. The data group of the first process condition may include a plurality of processing methods, and may include a predetermined single processing method.

For example, when the average waveform signal obtained by the above-described extraction method is processed as the first evaluation data by the first data obtaining unit 62, the first data obtaining unit 62 determines to output the average waveform signal to the regression analysis unit 620, and further determines the processing method to be performed to the average waveform signal by the regression analysis unit 620.

The regression analysis unit 620 to which the average waveform signal has been input from the first extracting unit 61 obtains, for example, the blood glucose level from the average waveform signal using the calibration model indicating the correlation between the measured value and the pulse wave signal, and outputs the blood glucose level as the first data.

The calibration model is established based on a result of an analysis performed by, for example, a regression analysis having the preliminarily measured average waveform pulse wave as an explanatory variable and the measured value of the biometric information as an objective variable. For the calibration model, the first data can be measured by storing a preliminarily established general-purpose calibration model in the storing unit or the like. The establishment of the calibration model is necessary in some cases, for example, when calibration is regularly performed or when the calibration model is re-established when the user changes.

For example, in the estimation of the biometric information with difficulty in observing an abnormal value and thus having difficulty in collecting data of the abnormal value, such as a blood carbon dioxide concentration, the regression analysis unit 620 may estimate the abnormal value of the biometric information from the degree of difference between the average waveform signal input from the first extracting unit 61 and the calibration model.

The regression analysis unit 620 includes a plurality of calibration models, and which calibration model is used to the input average waveform data is determined depending on the processing method determined by referring to the first process condition by the first data obtaining unit 62. For example, when the filter processing unit 610 selects the blood glucose level as the biometric information desired to be obtained, the first data obtaining unit 62 outputs the average waveform signal input from the first extracting unit 61 to the regression analysis unit 620. The regression analysis unit 620 performs an analysis by the regression analysis having the preliminarily measured waveform data of the pulse wave as an explanatory variable and the measured value of the blood glucose level as an objective variable, and determines to obtain the first data from the input average waveform data using a calibration model established based on the analysis result.

For example, when the filter processing unit 610 determines the extraction condition of the signal based on the age of the user as the external factor, the first data obtaining unit 62 outputs the average waveform signal input from the first extracting unit 61 to the regression analysis unit 620 by referring to the first process condition associated with the first extraction condition, performs an analysis by the regression analysis having the preliminarily measured waveform data of the pulse wave as an explanatory variable and the measured value of the blood glucose level of a user close to the age of the user as an objective variable by the regression analysis unit 620, and determines to obtain the first data from the input average waveform data using a calibration model established based on the analysis result. Accordingly, the processing method appropriate for the extraction method can be determined.

Next, in a second extraction condition determining step S14, the second extracting unit 63 inputs the pulse wave signal input from the pulse wave signal obtaining unit 60 to the filter processing unit 630. Then, the filter processing unit 630 determines the extraction method performed to the input pulse wave signal by referring to the second extraction condition.

The second extraction condition is a data group including a list of extraction methods for extracting the second evaluation data from the pulse wave signal. The data group of the second extraction condition may include a plurality of extraction methods, and may include a predetermined single extraction method. The second extraction condition may be the same as the first extraction condition.

The filter processing unit 630 determines the extraction method performed to the pulse wave signal by the second extracting unit 63 from the second extraction conditions depending on the state of the obtained pulse wave signal, the biometric information desired to be obtained, and an external factor. The external factor includes, for example, at least one of the user's information such as an age, a gender, a case history, lifestyle habits, a health condition, and genetic information of the user, and environment information such as a temperature and a humidity. The filter processing unit 630 may determine the extraction method performed to the pulse wave signal by the second extracting unit 63 depending on the contents of the first data obtained by the first data obtaining unit 62. For example, when the accuracy of the blood glucose level of the first data obtained by the first data obtaining unit 62 is low, the filter processing unit 630 determines the extraction method not performed by the first extracting unit 61. For example, when the blood glucose level is obtained as the first data, to obtain the blood pressure as the second data, the filter processing unit 630 may determine the extraction condition of performing a trimming with a certain sampling number for making the time width of the divided waveform signal uniform by the normalization unit 633. Accordingly, the second data can be obtained corresponding to the change and the feature of the first data, thus allowing obtaining the evaluation result with high accuracy. The filter processing unit 630 may determine the processing method performed to the second evaluation data by the second data obtaining unit 64 depending on the contents of the first data obtained by the first data obtaining unit 62. As a specific method, one similar to a method in which the second data obtaining unit 64 described later determines the processing method performed to the second evaluation data depending on the contents of the first data obtained by the first data obtaining unit 62 may be employed.

The filter processing unit 630 may determine the extraction method performed to the pulse wave signal by the second extracting unit 63 depending on the extraction method of the first evaluation data obtained by the first data obtaining unit 62. For example, when the differentiation is performed by the differentiation unit 611 in obtaining the first evaluation data, the filter processing unit 630 determines not to perform the differentiation by the differentiation unit 631. Accordingly, the optimal second data can be obtained accompanied by the change of the first data.

Next, in a second extracting step S15, the second extracting unit 63 extracts second evaluation data from the pulse wave signal input from the pulse wave signal obtaining unit 60. The second evaluation data is waveform data extracted from the pulse wave signal by the second extracting unit 63 for performing the process by the second data obtaining unit 64. The second evaluation data is waveform data, for example, formed by processing the pulse wave signal into a waveform of one cycle or the like by at least any of a filtering process, a differential process, a normalization process, and an averaging process.

First, the filter processing unit 630 performs the filtering process on the pulse wave signal input from the pulse wave signal obtaining unit 60, and then, for example, when the blood glucose level is obtained as the biometric information, based on the extraction method determined in the second extraction condition determining step S14, the filter processing unit 630 outputs the pulse wave signal to the differentiation unit 631.

Next, the differentiation unit 631 determines whether to differentiate the pulse wave signal input from the filter processing unit 630 or not based on the extraction method determined in the second extraction condition determining step S14, and after performing the process, outputs the pulse wave signal to the dividing unit 632.

Next, the dividing unit 632 divides each of a plurality of waveform signals input from the differentiation unit 631 into divided waveform data of one cycle. Then, the dividing unit 632 outputs the divided waveform data to the normalization unit 633.

The normalization unit 633 performs normalization for making time widths of a plurality of divided waveform signals input from the dividing unit 632 uniform, obtains an average waveform signal that is an average of the plurality of divided waveform signals, and performs normalization with a maximum value of 1 and a minimum value of 0 of the average waveform signal. At this time, the second extracting unit 63 obtains the average waveform signal as the second evaluation data. Then, the normalization unit 633 outputs the normalized average waveform signal to the second data obtaining unit 64.

Next, the biometric information computing system 100 transitions to a second data obtaining step S16, refers to the second process condition associated with the second extraction condition, processes the second evaluation data input from the second extracting unit 63 by the second data obtaining unit 64, thus obtaining the second data.

The second process condition is a data group including the method associated with the second extraction condition for the process performed by the second data obtaining unit 64 to the second evaluation data input from the second extracting unit 63. The second data obtaining unit 64 determines the processing method from the second process condition. The data group of the second process condition may include a plurality of processing methods, and may include a predetermined single processing method. The second process condition may be the same as the first process condition.

The second data obtaining unit 64 may determine the processing method of the second evaluation data performed by the second data obtaining unit 64 corresponding to the processing method of the first data. For example, when the processing method of obtaining the first data from the average waveform signal using the calibration model indicating the correlation between the measured value measured from a user of a young age group and the pulse wave signal is performed, the second data obtaining unit 64 may obtain the second data from the average waveform signal using a calibration model more appropriate for the user corresponding to the above-described processing method. Accordingly, a plurality of pieces of biometric information obtained by different processing methods can be obtained, thus allowing the more multifaceted evaluation with high accuracy.

The second data obtaining unit 64 may determine the processing method corresponding to the contents of the first data by referring to the second process condition. For example, when it is found that a user has a tendency of hypoglycemia from the first data, the second data obtaining unit 64 may determine a processing method of obtaining the blood glucose level using a calibration model indicating a correlation between the measured value of the blood glucose level of the user of hypoglycemia and the pulse wave signal as the processing method of the second evaluation data performed by the second data obtaining unit 64. Since a case where the obtainable value of the blood glucose level has a large error is assumed, a classification into a hypoglycemia zone, a normal blood glucose zone, a hyperglycemia zone, and a very hyperglycemia zone is performed from the contents of the first data, and by using a calibration model corresponding to the blood glucose zone, the accuracy of the blood glucose level obtained as the second data can be substantially improved. Additionally, whether the user is diabetic or not may be determined from the contents of the first data, and the processing method of the second evaluation data performed by the second data obtaining unit 64 may be determined depending on the result. Accordingly, the process of the second evaluation data is determined following the change of the first data, thereby allowing the evaluation with higher accuracy.

The processing method can be classified also depending on, for example, the contents of the calibration model used in the process of obtaining the first data from the average waveform signal by the regression analysis unit 620. For example, the processing method of obtaining the first data from the average waveform signal using the calibration model indicating the correlation between the measured value measured from a user of a young age group and the pulse wave signal and the processing method of obtaining the first data from the average waveform signal using the calibration model indicating the correlation between the measured value measured from a user of a different age group and the pulse wave signal are mutually different processing methods.

The second data obtaining unit 64 may refer to the classification pattern of the first evaluation data and determine the classification result of the first evaluation data as the first data, and refer to the above-described first data and determine the processing method of the second evaluation data performed by the second data obtaining unit 64. Accordingly, for example, it is allowed to classify the feature of the signal using the acceleration pulse wave that facilitates the classification, and then perform the evaluation with high accuracy using the velocity pulse wave that can suppress the false detection. Accordingly, the process of the second evaluation data more appropriate for the user can be determined following the change of the first data.

The classification pattern means a classification table for classifying the signal into two or more groups corresponding to the feature of the waveform of the signal, and for example, the classification pattern of the acceleration pulse wave illustrated in Fig. 12 is used.

When the first evaluation data based on the acceleration pulse wave obtained by differentiating the pulse wave signal by the differentiation unit 611 is input, the first data obtaining unit 62 determines, for example, which pattern of Fig. 12 the first evaluation data corresponds to, and determines the classification pattern. For example, when the inflection point ***b*** of the input first evaluation data is smaller than the inflection point **d** and further the inflection point **c** ≥ 0.5 is met, the pattern A is determined as the classification pattern of the first evaluation data.

Since the appropriate calibration model is different for each classification of the acceleration pulse wave, the second data obtaining unit 64 refers to the classification pattern of the first evaluation data to determine the classification result of the first evaluation data as the first data, and refers to the above-described first data to determine the processing method of the second evaluation data performed by the second data obtaining unit 64, thus allowing achieving the appropriate processing method for each user. For example, when the result in which the first evaluation data is classified into the pattern A is determined as the first data, the second data obtaining unit 64 performs an analysis by the regression analysis having the preliminarily measured waveform data of the pattern A of the pulse wave as an explanatory variable and the measured value of the biometric information as an objective variable by the regression analysis unit 640, and determines to obtain the second data from the input average waveform data using a calibration model established based on the analysis result.

Instead of the above-described classification pattern of the acceleration pulse wave, for example, the classification pattern of the velocity pulse wave illustrated in Fig. 13 may be used. By using this as described above, the first data obtaining unit 62 can determine the classification pattern even when the first evaluation data based on the velocity pulse wave without the differentiation of the pulse wave signal by the differentiation unit 611 is input.

Next, in an optimal blood glucose level calculating step S17, the first data obtained by the first data obtaining unit 62 and the second data obtained by the second data obtaining unit 64 are input to the optimal blood glucose level calculating unit 65, thus obtaining optimal biometric information from the first data and the second data. For example, with a first blood glucose level that is the blood glucose level obtained as the first data and a second blood glucose level that is the blood glucose level obtained as the second data, an optimal blood glucose level is calculated from the first blood glucose level and the second blood glucose level. For the calculation method of the biometric information having the optimal value, for example, the first data and the second data may be weighted based on the respective measurement accuracies, thereby calculating the biometric information having the optimal value from a plurality of first data and a plurality of second data based on the weighting. Other examples include a method in which a blood glucose level obtained by another sensor or the like is used as a reference value, plot graphs of the reference value and the first data and the second data are made, and data indicating a satisfactory value on the error grid in the two plot graphs is output, a method in which a plurality of first data and a plurality of second data are obtained, and the data with small variation is output, and a method in which whether the first data and the second data are included in a predetermined allowable range or not is evaluated, and the included data is output.

Here, in the waveform signal of the pulse wave, the obtainable value of the biometric information has the variation depending on the extraction condition or the processing method in some cases. For example, when the case where the differentiated pulse wave is processed as the extraction condition is compared with the case where the pulse wave without differentiation is processed as the extraction condition, it is concerned that the obtainable value of the biometric information has the variation. That is, for obtaining the biometric information with sufficient accuracy using the waveform signal of the pulse wave, it is necessary to multidirectionally evaluate the waveform signal of the pulse wave from a plurality of pieces of biometric information simultaneously output through multiple kinds of extraction conditions or the like.

On the other hand, in the prior art, it is not disclosed that, to one waveform signal of the pulse wave, a plurality of pieces of biometric information are simultaneously output through multiple kinds of extraction conditions or the like. Therefore, in the prior art, since the variation occurs in the obtainable biometric information depending on the extraction condition or the like, it is concerned that the sufficient accuracy is not obtained.

In contrast, the biometric information computing system 100 according to this embodiment includes, for example, the pulse wave signal obtaining step S10 of obtaining the velocity pulse wave as the pulse wave signal, the first extracting step S12 of referring to the first extraction condition and extracting the first evaluation data based on the pulse wave signal, the first data obtaining step S13 of referring to the first process condition associated with the first extraction condition and obtaining the first data to the first evaluation data, the second extracting step S15 of referring to the second extraction condition and extracting the second evaluation data based on the pulse wave signal, and the second data obtaining step S16 of referring to the second process condition associated with the second extraction condition and obtaining the second data to the second evaluation data. The second extracting step S15 determines any of the plurality of extraction methods included in the second extraction condition to be referred to or any of the plurality of processing methods included in the second process condition corresponding to the first data.

That is, according to this embodiment, by the first extracting step S12 referring to the first extraction condition, the first evaluation data based on the pulse wave signal is extracted, and further, by the first data obtaining step S13 referring to the first process condition associated with the first extraction condition, the first data to the first evaluation data is obtained. The biometric information computing system 100 extracts the second evaluation data based on the pulse wave signal by the second extraction S15 referring to the second extraction condition, and further, obtains the second data to the second evaluation data by the second data obtaining step S16 referring to the second process condition associated with the second extraction condition. Accordingly, from one input pulse wave signal, a plurality of pieces of biometric information to which different extraction methods and processing methods have been performed can be simultaneously obtained. With the plurality of pieces of biometric information, the evaluation result with high accuracy can be obtained by the multifaceted evaluation of the biometric information with one pulse wave signal.

According to this embodiment, corresponding to the first data obtained by the first data obtaining step S13, the second extracting step S15 determines any of the plurality of extraction methods included in the second extraction condition to be referred to or any of the plurality of processing methods included in the second process condition. Therefore, it is allowed to determine the extraction method for the optimal pulse wave signal from the contents of the first data and obtain the second data corresponding to the change or the feature of the first data. Accordingly, the evaluation result corresponding to the feature of the user can be obtained with high accuracy.

According to this embodiment, the second extracting step S15 determines any of the plurality of extraction methods included in the second extraction condition to be referred to based on the extraction method of the first evaluation data extracted by the first extracting step S12. Accordingly, the second extracting step S15 can determine the more appropriate extraction method from the extraction methods of the first evaluation data, and obtain a plurality of pieces of biometric information that are more appropriate for the pulse wave signal and extracted by different methods, thus allowing obtaining the evaluation result with higher accuracy by the multifaceted evaluation.

According to this embodiment, the second data obtaining step S16 determines any of the plurality of processing methods included in the second process condition to be referred to corresponding to the processing method of the first data determined by the first data obtaining step S13. Accordingly, the second data obtaining step S16 can determine the optimal processing method of the second evaluation data from the processing method of the first data, obtain a plurality of pieces of biometric information that are more appropriate for the pulse wave signal and processed by different methods, and obtain the evaluation result with higher accuracy by the more multifaceted evaluation.

According to this embodiment, the first extracting step S12 differentiates the pulse wave signal and extracts the first evaluation data, the first data obtaining step S13 refers to the classification pattern of the first evaluation data and obtains the classification result of the first evaluation data as the processing method of the first data, and the second extracting step S15 extracts the second evaluation data without differentiating the pulse wave signal. Accordingly, the classification can be performed with the acceleration pulse wave appropriate for the classification as the first evaluation data, and the process can be performed with the velocity pulse wave that can suppress the false detection as the second evaluation data, thus allowing obtaining the evaluation result with higher accuracy by the multifaceted evaluation.

### (Eighth Embodiment: Biometric Information Computing System 100)

Next, an example of a biometric information computing system 100 in the eighth embodiment will be described. The difference between the above-described embodiments and the eighth embodiment is that an electronic device 2 performs a different process. For the contents similar to the above-described embodiments, the explanation will be omitted.

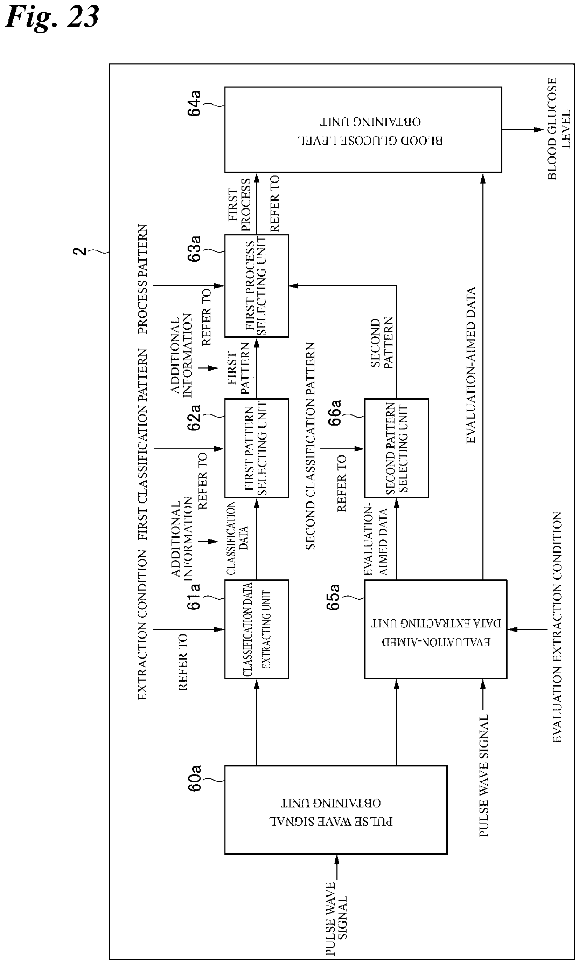

Fig. 23 illustrates a sequence for achieving a biometric information computing program of the electronic device 2. The electronic device 2 includes a pulse wave signal obtaining unit 60a, a classification data extracting unit 61a and an evaluation-aimed data extracting unit 65a connected to the pulse wave signal obtaining unit 60a, a first pattern selecting unit 62a connected to the classification data extracting unit 61a, a second pattern selecting unit 66a connected to the evaluation-aimed data extracting unit 65a, a first process selecting unit 63a connected to the first pattern selecting unit 62a and the second pattern selecting unit 66a, and a blood glucose level obtaining unit 64a connected to the first process selecting unit 63a and the evaluation-aimed data extracting unit 65a.

The pulse wave signal obtaining unit 60a obtains a pulse wave signal transmitted from the sensor 5, the server 4, and another electronic device 2 via the communications network 3. The pulse wave signal obtaining unit 60a outputs the obtained pulse wave signal to the classification data extracting unit 61a and the evaluation-aimed data extracting unit 65a. When extracting the pulse wave signal by the evaluation-aimed data extracting unit 65a is not required, the pulse wave signal obtaining unit 60a may output the pulse wave signal to the blood glucose level obtaining unit 64a.

The classification data extracting unit 61a refers to an extraction condition, and extracts classification data based on the pulse wave signal input from the pulse wave signal obtaining unit 60a. The classification data extracting unit 61a outputs the extracted classification data to the first pattern selecting unit 62a.

The first pattern selecting unit 62a refers to a first classification pattern, and selects a first pattern to the classification data input from the classification data extracting unit 61a. The first pattern selecting unit 62a outputs the selected first pattern to the first process selecting unit 63a.

The evaluation-aimed data extracting unit 65a refers to an evaluation extraction condition, and extracts evaluation-aimed data based on the pulse wave signal input from the pulse wave signal obtaining unit 60a. The evaluation-aimed data extracting unit 65a outputs the extracted evaluation-aimed data to the second pattern selecting unit 66a and the blood glucose level obtaining unit 64a.

The second pattern selecting unit 66a refers to a second classification pattern, and selects a second pattern to the evaluation-aimed data input from the evaluation-aimed data extracting unit 65a. The second pattern selecting unit 66a outputs the selected second pattern to the first process selecting unit 63a.

The first process selecting unit 63a refers to a preliminarily obtained process pattern, and selects a first process based on the first pattern input from the first pattern selecting unit 62a and the second pattern input from the second pattern selecting unit 66a. The first process selecting unit 63a outputs the selected first process on the blood glucose level obtaining unit 64a.

The blood glucose level obtaining unit 64a refers to the first process input from the first process selecting unit 63a, and obtains a blood glucose level to the evaluation-aimed data input from the evaluation-aimed data extracting unit 65a.

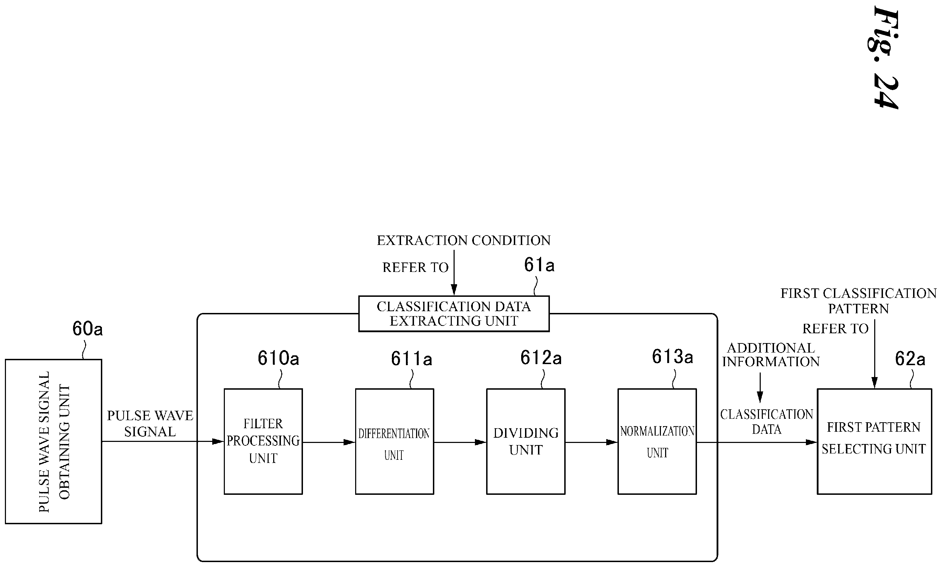

Fig. 24 illustrates an example of a specific configuration of the classification data extracting unit 61a. The classification data extracting unit 61a includes a filter processing unit 610a connected to the pulse wave signal obtaining unit 60a, a differentiation unit 611a connected to the filter processing unit 610a, a dividing unit 612a connected to the differentiation unit 611a, a normalization unit 613a connected to the dividing unit 612a.
the classification data extracting unit 61a does not necessarily use all of the extraction methods included in the classification data extracting unit 61a for obtaining one classification data, but extracts the classification data from the pulse wave signal with at least one extraction method determined depending on the extraction condition.

The filter processing unit 610a performs a filtering process on the obtained pulse wave signal. In the filter processing unit 610a, a bandpass filter of, for example, 0.5 to 5 Hz is used for filtering, but it is not limited to this. The filter processing unit 610a refers to the extraction condition, and determines an extraction method for extracting the classification data from the obtained pulse wave signal. The filter processing unit 610a outputs the filter-processed pulse wave signal to differentiation unit 611a.

The differentiation unit 611a differentiates the pulse wave signal input from the filter processing unit 610a. When the filter processing unit 610a determines the differential process on be necessary, the differentiation unit 611a performs a differential process on the input pulse wave signal. The differentiation unit 611a outputs the processed pulse wave signal to the dividing unit 612a.

The dividing unit 612a divides each of a plurality of waveform signals input from the differentiation unit 611a into divided waveform data of integer cycles. In the dividing unit 612a, while the integer cycle is set to one cycle in this embodiment, the integer cycle may be multiple cycles. The dividing unit 612a outputs the divided waveform data to the normalization unit 613a.

The normalization unit 613a performs normalization for making time widths of a plurality of divided waveform signals input from the dividing unit 612a uniform, obtains an average waveform signal that is an average of the plurality of divided waveform signals, and performs normalization with a maximum value of 1 and a minimum value of 0 of an amplitude of the average waveform signal, thus obtaining the classification data. The normalization unit 613a outputs the obtained classification data to the first pattern selecting unit 62a.

The normalization unit 613a may perform trimming of the plurality of divided waveform signals input from the dividing unit 612a with a certain time width or a certain sampling number for making the time widths of the divided waveform signals uniform. The processing method for making the time width uniform by the normalization unit 613a is determined by the filter processing unit 610a.

While the normalization unit 613a requires a plurality of divided waveform signals in obtaining the average waveform signal, the number of the required divided waveform signals is determined by the filter processing unit 610a.

The first pattern selecting unit 62a refers to the preliminarily obtained first classification pattern, and classifies the classification data input from the normalization unit 613a into the first pattern. The first pattern selecting unit 62a outputs the classified first pattern to the first process selecting unit 63a.

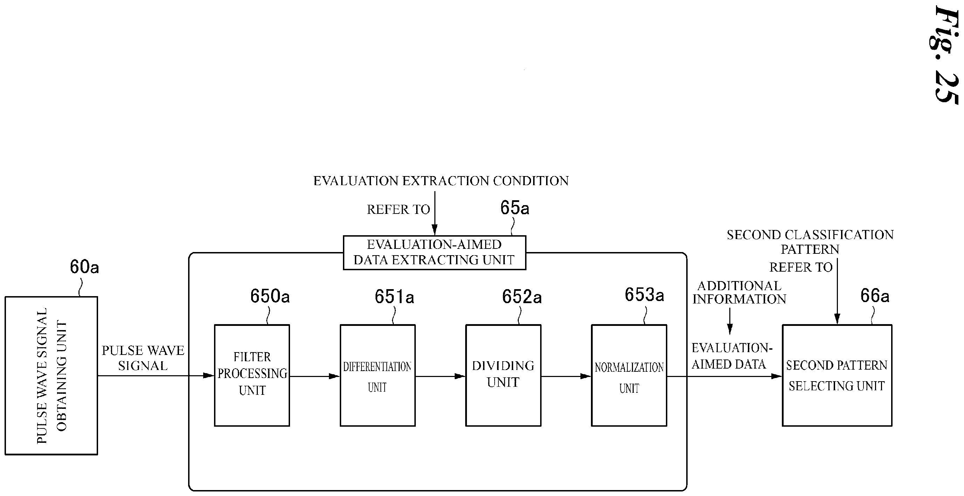

Fig. 25 illustrates an example of a specific configuration of the evaluation-aimed data extracting unit 65a. The evaluation-aimed data extracting unit 65a includes a filter processing unit 650a connected to the pulse wave signal obtaining unit 60a, a differentiation unit 651a connected to the filter processing unit 650a, a dividing unit 652a connected to the differentiation unit 651a, a normalization unit 653a connected to the dividing unit 652a. The evaluation-aimed data extracting unit 65a is configured with the same one as the classification data extracting unit 61a, and while the classification data extracting unit 61a refers to the extraction condition, the evaluation-aimed data extracting unit 65a refers to the evaluation extraction condition instead of the extraction condition.

The filter processing unit 650a performs a filtering process on the obtained pulse wave signal. In the filter processing unit 650a, a bandpass filter of, for example, 0.5 to 5 Hz is used for filtering, but it is not limited to this. The filter processing unit 650a refers to the evaluation extraction condition, and determines an extraction method for extracting the evaluation-aimed data from the obtained pulse wave signal. The filter processing unit 650a outputs the filter-processed pulse wave signal to the differentiation unit 651a.

The differentiation unit 651a differentiates the pulse wave signal input from the filter processing unit 650a. When the filter processing unit 650a determines the differential process to be necessary, the differentiation unit 651a performs a differential process on the input pulse wave signal. The differentiation unit 651a outputs the processed pulse wave signal to the dividing unit 652a.

The dividing unit 652a divides each of a plurality of waveform signals input from the differentiation unit 651a into divided waveform data of integer cycles. In the dividing unit 652a, while the integer cycle is set to one cycle in this embodiment, the integer cycle may be multiple cycles. The dividing unit 652a outputs the divided waveform data to the normalization unit 653a.

The normalization unit 653a performs normalization for making time widths of a plurality of divided waveform signals input from the dividing unit 652a uniform, obtains an average waveform signal that is an average of the plurality of divided waveform signals, and performs normalization with a maximum value of 1 and a minimum value of 0 of an amplitude of the average waveform signal, thus obtaining the evaluation-aimed data. The normalization unit 653a outputs the obtained evaluation-aimed data to the second pattern selecting unit 66a.

The normalization unit 653a may perform trimming of the plurality of divided waveform signals input from the dividing unit 652a with a certain time width or a certain sampling number for making the time widths of the divided waveform signals uniform. The method for making the time width uniform by the normalization unit 653a is determined by the filter processing unit 650a.

While the normalization unit 653a requires a plurality of divided waveform signals in obtaining the average waveform signal, the number of the required divided waveform signals is determined by the filter processing unit 650a.

The second pattern selecting unit 66a refers to the preliminarily obtained second classification pattern, and classifies the evaluation-aimed data input from the normalization unit 653a into the second pattern. The second pattern selecting unit 66a outputs the classified second pattern to the first process selecting unit 63a.

The first process selecting unit 63a refers to a preliminarily obtained process pattern, and selects the first process from the first pattern input from the first pattern selecting unit 62a and the second pattern input from the second pattern selecting unit 66a. The first process selecting unit 63a outputs the selected first process on the blood glucose level obtaining unit 64a.

The biometric information obtained by the biometric information computing system 100 includes the blood glucose level, and as other examples, may include the blood pressure, the blood oxygen saturation, the blood carbon dioxide concentration, the vascular age, the degree of diabetes, and the like. The biometric information computing system 100 may obtain, for example, information other than above having a correlation with the pulse wave signal as the biometric information.

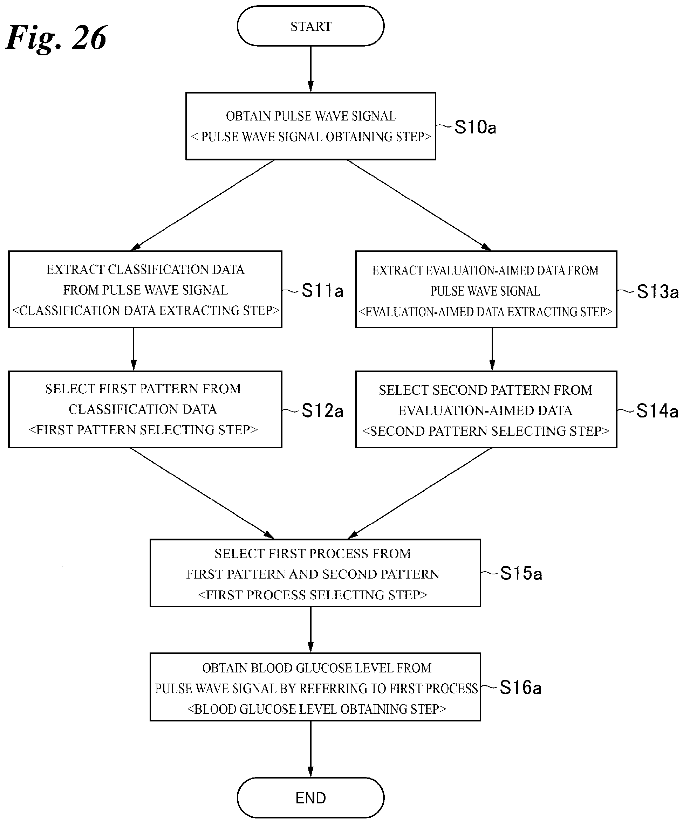

Next, an example of the operation of the biometric information computing system 100 according to this embodiment will be described. Fig. 26 is a flowchart illustrating an example of the operation of the biometric information computing system 100 according to this embodiment.

First, in a pulse wave signal obtaining step S 10a, the obtaining unit 50 obtains the pulse wave signal, and outputs the pulse wave signal to the communication I/F 51 via the internal bus 54. For example, instead of the pulse wave signal obtained by the obtaining unit 50, the pulse wave signal obtaining step S10a may output the pulse wave signal stored in the memory 52 to the communication I/F 51 via the internal bus 54. The pulse wave signal is measured using, for example, an FBG sensor.

Next, the communication I/F 51 to which the pulse wave signal has been input from the obtaining unit 50 transmits the pulse wave signal to the pulse wave signal obtaining unit 60a via the communications network 3. At this time, instead of the pulse wave signal obtained by the obtaining unit 50, the pulse wave signal stored in the server 4 may be transmitted to the pulse wave signal obtaining unit 60a.

Next, the pulse wave signal obtaining unit 60a to which the pulse wave signal has been transmitted via the communications network 3 outputs the pulse wave signal to the classification data extracting unit 61a and the evaluation-aimed data extracting unit 65a.

The classification data extracting unit 61a refers to the extraction condition, and extracts the classification data from the pulse wave signal input from the pulse wave signal obtaining unit 60a. The classification data is waveform data extracted by the classification data extracting unit 61a for classifying the pulse wave signal by the first pattern selecting unit 62a. The classification data is waveform data, for example, formed by processing the pulse wave signal into a waveform of one cycle or the like by at least any of a filtering process, a differential process, a normalization process, and an averaging process.

The filter processing unit 610a refers to the extraction condition, and determines the extraction method performed to the pulse wave signal by the classification data extracting unit 61a depending on the state of the obtained pulse wave signal and/or additional information. The additional information indicates the information on the user, includes, for example, information on an age, a gender, a case history, lifestyle habits, a health condition, medication information, a degree of arteriosclerosis, genetic information, or the like of the user, and additionally, includes at least one of environment information such as a temperature, a humidity, or an acceleration measured by an acceleration sensor attached to the sensor 5.

For example, the filter processing unit 610a instructs the differentiation unit 611a to differentiate the pulse wave signal input from the filter processing unit 610a.

First, the classification data extracting unit 61a outputs the pulse wave signal input from the pulse wave signal obtaining unit 60a to the filter processing unit 610a.

Next, the filter processing unit 610a performs the filtering process on the pulse wave signal input from the pulse wave signal obtaining unit 60a, and then, outputs the pulse wave signal to the differentiation unit 611a.

Next, the differentiation unit 611a determines whether to differentiate the pulse wave signal input from the filter processing unit 610a or not based on the filter processing unit 610a, and after performing the process, outputs the pulse wave signal to the dividing unit 612a.

The reason for determining whether to differentiate the pulse wave signal by the differentiation unit 611a or not based on the filter processing unit 610a is to determine the appropriate first process depending on the obtained pulse wave signal because the feature of the obtained classification data differs depending on whether to differentiate the pulse wave signal or not.

Next, the dividing unit 612a divides each of a plurality of waveform signals input from the differentiation unit 611a into divided waveform data of one cycle to perform averaging. Then, the dividing unit 612a outputs the divided waveform data to the normalization unit 613a.

The normalization unit 613a performs normalization of the horizontal axis for making time widths of a plurality of divided waveform signals input from the dividing unit 612a uniform, obtains an average waveform signal that is an average of the plurality of divided waveform signals, and performs normalization of the vertical axis with a maximum value of 1 and a minimum value of 0 of the average waveform signal, thus obtaining the classification data. Then, the normalization unit 613a outputs the classification data to the first pattern selecting unit 62a.

The reason for performing the normalization process of the horizontal axis for making the time width uniform by the normalization unit 613a is that since a difference is significantly generated in a terminating end side of the pulse wave, by deleting this part, the main body part of the pulse wave is obtained as the analysis object. The reason for performing the normalization process of the vertical axis with the maximum value of 1 and the minimum value of 0 of the average waveform signal is to average a variation of a pressing pressure in attaching the FBG sensor to the measurement position or a variation of measurement data due to the position shift of the FBG sensor in the measurement, and is to improve the accuracy of the correlation between the pulse wave signal and the measured value of the biometric information by reducing the noise caused by the variation in the measurement.

Next, a first pattern selecting step S12a refers to the first classification pattern, and classifies the classification data input from the classification data extracting unit 61a by the first pattern selecting unit 62a, thus obtaining the first pattern. As the classification pattern, for example, similarly to the above-described embodiment, the classification pattern of acceleration pulse wave as illustrated in Fig. 12 or the like is used.

When the classification data based on the acceleration pulse wave obtained by differentiating the pulse wave signal by the differentiation unit 611a is input, the first pattern selecting unit 62a determines, for example, which pattern of Fig. 12 the classification data corresponds to, and determines the first pattern.

Next, in an evaluation-aimed data extracting step S13a, the evaluation-aimed data extracting unit 65a refers to the evaluation extraction condition, and extracts the evaluation-aimed data from the pulse wave signal input from the pulse wave signal obtaining unit 60a. While the extraction method performed to the pulse wave signal by the evaluation-aimed data extracting unit 65a is approximately similar to the extraction method performed to the pulse wave signal by the classification data extracting unit 61a, there is a difference in that while the classification data extracting unit 61a refers to the extraction condition, the evaluation-aimed data extracting unit 65a refers to the evaluation extraction condition instead of the extraction condition. The evaluation-aimed data extracting unit 65a may receive a pulse wave signal different from the pulse wave signal input to the classification data extracting unit 61a from the pulse wave signal obtaining unit 60a from the pulse wave signal obtaining unit 60a, and extract the evaluation-aimed data from the above-described pulse wave signal. The evaluation-aimed data is waveform data extracted by the evaluation-aimed data extracting unit 65a for classifying the pulse wave signal by the second pattern selecting unit 66a or obtaining the biometric information by the blood glucose level obtaining unit 64a. The evaluation-aimed data is waveform data, for example, formed by processing the pulse wave signal into a waveform of one cycle or the like by at least any of a filtering process, a differential process, a normalization process, and an averaging process.

The filter processing unit 650a refers to the evaluation extraction condition, and determines the extraction method performed to the pulse wave signal by the evaluation-aimed data extracting unit 65a depending on the state of the obtained pulse wave signal and/or additional information. For the extraction method, for example, when a high value of the above-described acceleration is measured as the additional information, a bandpass filter having a narrower passband may be used for the filter processing unit 650a.

The filter processing unit 650a determines the extraction method performed to the pulse wave signal by the evaluation-aimed data extracting unit 65a also depending on the extraction method of the classification data. For example, when the extraction method of performing the differentiation has been determined to the differentiation unit 611a, the filter processing unit 650a instructs the differentiation unit 651a not to perform the differentiation of the pulse wave signal input from the filter processing unit 650a. Accordingly, it is allowed to perform the classification using the acceleration pulse wave that facilitates the classification as the classification data, select the first pattern to the classification data, and determine the first process on the first pattern, and allowed to obtain the blood glucose level to the evaluation-aimed data using the velocity pulse wave that easily suppresses the false detection as the evaluation-aimed data, thus allowing the evaluation with higher accuracy.

First, the evaluation-aimed data extracting unit 65a outputs the pulse wave signal input from the pulse wave signal obtaining unit 60a to the filter processing unit 650a.

Next, the filter processing unit 650a performs the filtering process on the pulse wave signal input from the pulse wave signal obtaining unit 60a, and then outputs the pulse wave signal to the differentiation unit 651a.

Next, the differentiation unit 651a determines whether to differentiate the pulse wave signal input from the filter processing unit 650a or not based on the filter processing unit 650a, and after performing the process, outputs the pulse wave signal to the dividing unit 652a.

Next, the dividing unit 652a divides each of a plurality of waveform signals input from the differentiation unit 651a into divided waveform data of one cycle to perform averaging. Then, the dividing unit 652a outputs the divided waveform data to the normalization unit 653a.

The normalization unit 653a performs normalization of the horizontal axis for making time widths of a plurality of divided waveform signals input from the dividing unit 652a uniform, obtains an average waveform signal that is an average of the plurality of divided waveform signals, and performs normalization of the vertical axis with a maximum value of 1 and a minimum value of 0 of the average waveform signal, thus obtaining the evaluation-aimed data. Then, the normalization unit 653a outputs the evaluation-aimed data to the second pattern selecting unit 66a.

The reason for performing the normalization process of the horizontal axis for making the time width uniform by the normalization unit 653a is that since a difference is significantly generated in a terminating end side of the pulse wave, by deleting this part, the main body part of the pulse wave is obtained as the analysis object. The reason for performing the normalization process of the vertical axis with the maximum value of 1 and the minimum value of 0 of the average waveform signal is to average a variation of a pressing pressure in attaching the FBG sensor to the measurement position or a variation of measurement data due to the position shift of the FBG sensor in the measurement, and is to improve the accuracy of the correlation between the pulse wave signal and the measured value of the biometric information by reducing the noise caused by the variation in the measurement.

Next, a second pattern selecting step S14a refers to the second classification pattern, and classifies the evaluation-aimed data input from the evaluation-aimed data extracting unit 65a by the second pattern selecting unit 66a, thus obtaining the second pattern.

The second pattern selecting unit 66a determines, similarly to the first pattern selecting unit 62a, for example, which pattern the input evaluation-aimed data and additional information correspond to, and determines the second pattern.

Similarly to the first pattern selecting unit 62a, the second pattern selecting unit 66a may weight each of the classification results from the input plurality of classification data and additional information, and set the proportion of a plurality of patterns to the second pattern.

Next, in a first process selecting step S 15a, the first process selecting unit 63a refers to a preliminarily obtained process pattern, selects a first process on the first pattern input from the first pattern selecting unit 62a and the second pattern input from the second pattern selecting unit 66a, and outputs it to the blood glucose level obtaining unit 64a.

The process pattern is a data group including a sequence of processing methods performed to the evaluation-aimed data by the blood glucose level obtaining unit 64a to obtain the blood glucose level from the evaluation-aimed data. The first process selecting unit 63a determines the processing method performed to the evaluation-aimed data from the process pattern as the first process. The data group of the process pattern may include a plurality of processing methods. Examples of the process pattern include a processing method of obtaining the blood glucose level from the evaluation-aimed data using a calibration model indicating the correlation between the measured value and the pulse wave signal. The process pattern includes a processing method of, for example, estimating the abnormal value of the blood glucose level from the degree of difference between the input evaluation-aimed data and the calibration model.

The first process selecting unit 63a may select the processing method using the optimal calibration model for the first pattern input from the first pattern selecting unit 62a and the second pattern input from the second pattern selecting unit 66a as the first process, and output it to the blood glucose level obtaining unit 64a.

As a selection method of the first process, for example, an analysis may be performed by a regression analysis having waveform data of a pattern of the pulse wave most corresponding to the first pattern input from the first pattern selecting unit 62a and the second pattern input from the second pattern selecting unit 66a as an explanatory variable and the measured value of the blood glucose level as an objective variable, and a process pattern using a calibration model established based on the analysis result may be selected as a first process and output to the blood glucose level obtaining unit 64a. For example, when the pattern B is input as the first pattern, the analysis is performed with the regression analysis having the waveform data of the pattern B as the explanatory variable and the measured value of the blood glucose level as the objective variable, and the processing method using the calibration model established based on the analysis result is selected from the process pattern as the first process.

When the first pattern input from the first pattern selecting unit 62a and the second pattern input from the second pattern selecting unit 66a include a plurality of patterns, the first process selecting unit 63a may select a plurality of first processes using a plurality of calibration models corresponding to the respective patterns and output the plurality of first processes on the blood glucose level obtaining unit 64a.

The first process selecting unit 63a may select the first process on the input additional information. For example, when the age of the user of 40s is input as the additional information, the first process selecting unit 63a may select the processing method using the calibration model indicating the correlation between the measured value measured from the user of 40s and the pulse wave signal as the first process and output it to the blood glucose level obtaining unit 64a. For example, with the degree of arteriosclerosis or whether diabetes or not as the additional information, as described above, the first process selecting unit 63a may select the processing method using the calibration model indicating the correlation between the measured value measured from the user corresponding to the additional information and the pulse wave signal as the first process and output it to the blood glucose level obtaining unit 64a.

Next, in a blood glucose level obtaining step S 16a, the blood glucose level obtaining unit 64a refers to the first process input from the first process selecting unit 63a, and obtains the biometric information such as a blood glucose level to the evaluation-aimed data input from the evaluation-aimed data extracting unit 65a.

When a plurality of first processes are input from the first process selecting unit 63a, the blood glucose level obtaining unit 64a may obtain a plurality of blood glucose levels to the evaluation-aimed data based on the respective first processes. In this case, for example, an optimal blood glucose level may be calculated from the plurality of blood glucose levels. As a calculation method of the optimal blood glucose level, an average value of the plurality of blood glucose levels may be output as the optimal blood glucose level. For example, the blood glucose levels may be weighted based on the respective measurement accuracies, thereby calculating the optimal blood glucose level from the plurality of blood glucose levels based on the weighting. Other examples include a method in which a blood glucose level obtained by a first process indicating a satisfactory value on the error grid among the plurality of first processes obtaining the blood glucose levels is output as the optimal blood glucose level, a method in which a plurality of blood glucose levels are obtained using each of the first processes, and the blood glucose level obtained by the first process with small variation is output as the optimal blood glucose level, and a method in which whether the blood glucose level is included in a predetermined allowable range or not is evaluated, and the included blood glucose level is output as the optimal blood glucose level.

Here, in the waveform signal of the pulse wave, the value of blood glucose level obtainable from the waveform signal of the pulse wave has the variation due to the attribute such as a gender and an age of the user in some cases. For example, when the case where the pulse wave measured from a male in his 20s as a user is processed is compared with the case where the pulse wave measured from a female in her 50s as a user is processed, it is concerned that the variation occurs in the accuracy of the obtainable blood glucose level depending on the processing method and the sufficient accuracy is not obtained.

In contrast, the biometric information computing system 100 according to this embodiment includes the pulse wave signal obtaining step S10a of obtaining the velocity pulse wave as the pulse wave signal, the classification data extracting step S11a of extracting the classification data based on the pulse wave signal, the evaluation-aimed data extracting step S 13a of referring to the evaluation extraction condition and extracting the evaluation-aimed data based on the pulse wave signal by the extraction method different from the classification data, the first pattern selecting step S12a of referring to the first classification pattern including a plurality of preliminarily obtained first patterns and selecting one or more first patterns to the classification data, the first process selecting step S 15a of referring to the process pattern including a plurality of preliminarily obtained first processes and selecting one or more first processes on the first patterns, and the biometric information obtaining step (for example, blood glucose level obtaining step S 16a) of referring to the first process and obtaining the biometric information such as the blood glucose level from the evaluation-aimed data.

That is, according to this embodiment, the biometric information computing system 100 refers to the first process on the first pattern selected by the first process selecting step S 15a, and obtains the biometric information such as a blood glucose level from the evaluation-aimed data extracted by the evaluation-aimed data extracting step S 13a. Accordingly, since the optimal processing method to the pulse wave signal can be selected from the input pulse wave signal and the biometric information such as a blood glucose level can be obtained, the process of the pulse wave signal corresponding to the user attribute can be performed, thus allowing obtaining the evaluation result with high accuracy.

According to this embodiment, the biometric information computing system 100 extracts the acceleration pulse wave as the classification data by differentiating the pulse wave signal in the classification data extracting step S11a, and select the first pattern to the classification data. The evaluation-aimed data extracting step S13a extracts the velocity pulse wave as the evaluation-aimed data by not differentiating the pulse wave signal. Accordingly, since the feature of the pulse wave signal is classified using the acceleration pulse wave appropriate for the classification, and then the blood glucose level is measured using the velocity pulse wave that can suppress the false detection, the evaluation can be performed with high accuracy.

According to this embodiment, the first process selecting step S15a selects the first process on the first pattern selected by the first pattern selection means and the second pattern selected by the second pattern selecting steps S 12a, S 14a. Accordingly, since the more optimal processing method can be selected from a plurality of patterns that are obtained from one pulse wave signal and has been performed with different extraction methods, the accuracy is further improved.

According to this embodiment, the first pattern selecting step S12a selects the first pattern to the classification data and the additional information extracted by the classification data extracting step S11a. Accordingly, since the first pattern to the additional information can be selected, the accuracy is further improved.

According to this embodiment, the first process selecting step S15a selects the first process on the first pattern and the additional information extracted by the classification data extracting step S 11a. Accordingly, since the first process on the additional information can be selected, the accuracy is further improved.

While the embodiments of the present invention have been described, the embodiments have been presented as examples, and are not intended to limit the scope of the invention. The novel embodiments described herein can be embodied in a variety of other configurations. Various omissions, substitutions and changes can be made without departing from the gist of the invention. The embodiments and the modifications thereof are within the scope and the gist of the invention and within the scope of the inventions described in the claims and their equivalents.

### DESCRIPTION OF REFERENCE SIGNS

- 1:: Biometric information computing device
- 3:: Communications network
- 4:: Server
- 5:: Sensor
- 6:: Detecting unit
- 10:: Housing
- 11:: Obtaining unit
- 12:: Generating unit
- 13:: Output unit
- 14:: Storing unit
- 15:: Learning unit
- 50:: Obtaining unit
- 51:: Communication I/F
- 52:: Memory
- 53:: Instruction unit
- 54:: Internal bus
- 55:: Wristband
- 100:: Biometric information computing system
- 101:: CPU
- 102:: ROM
- 103:: RAM
- 104:: Storage unit
- 105:: I/F
- 106:: I/F
- 107:: I/F
- 108:: Input unit
- 109:: Display unit
- 110:: Internal bus
- S110:: Obtaining step
- S 120:: Generating step
- S 130:: Outputting step
- S 140:: Storing step
- S150:: Comprehensive evaluation step
- S 160:: Calculating step
- S170:: Updating step

## Claims

1. A biometric information computing system for evaluating biometric information of a user, comprising:
obtaining means that obtains first evaluation data and second evaluation data based on a pulse wave of the user;
a database that stores classification information generated using a plurality of training data, the training data being a pair of input data based on a preliminarily obtained training pulse wave and reference data including biometric information associated with the input data; and
generating means that generates a first evaluation result including first biometric information for the first evaluation data by referring to the database.

2. The biometric information computing system according to claim 1, wherein
the generating means includes generating a second evaluation result including second biometric information for the second evaluation data, and the second biometric information is different in kind from the first biometric information.

3. The biometric information computing system according to claim 2, further comprising
storage means that stores the first evaluation result and the second evaluation result.

4. The biometric information computing system according to claim 2, wherein
the obtaining means obtains the first evaluation data and the second evaluation data by performing mutually different kinds of processes on one pulse wave data corresponding to any of a velocity pulse wave and an acceleration pulse wave based on the pulse wave of the user.

5. The biometric information computing system according to claim 2, wherein
the classification information includes first classification information and second classification information generated using different kinds of the training data, and
the generating means includes:
generating the first evaluation result for the first evaluation data by referring to the first classification information; and
generating the second evaluation result for the second evaluation data by referring to the second classification information.

6. The biometric information computing system according to claim 2, comprising
comprehensive evaluation means that obtains additional information indicating a feature of the user and generates a comprehensive evaluation result comprehensively evaluating the feature of the user based on the first evaluation result, the second evaluation result, and the additional information.

7. The biometric information computing system according to claim 1, wherein
the classification information includes a plurality of pieces of attribute-based classification information calculated using the mutually different training data, and
the generating means includes:
selecting means that selects first classification information among the plurality of pieces of attribute-based classification information by referring to the second evaluation data; and
attribute-based generating means that generates the first evaluation result for the first evaluation data by referring to the first classification information.

8. The biometric information computing system according to claim 7, wherein the obtaining means includes:
obtaining data corresponding to a velocity pulse wave based on the pulse wave as the first evaluation data; and
obtaining data corresponding to an acceleration pulse wave based on the pulse wave as the second evaluation data.

9. The biometric information computing system according to claim 1, wherein the generating means includes:
selecting first classification information among the classification information based on a feature of the pulse wave; and
generating the first evaluation result for the first evaluation data by referring to the first classification information.

10. The biometric information computing system according to claim 3, comprising
calculating means that generates a comprehensive evaluation result comprehensively evaluating a feature of the user based on the first evaluation result and the second evaluation result stored by the storage means.

11. The biometric information computing system according to claim 3, wherein the storage means includes:
obtaining a determination result determined by the user for contents of the first evaluation result and the second evaluation result; and
mutually associating and storing the determination result, the first evaluation result, and the second evaluation result.

12. The biometric information computing system according to claim 11, comprising
updating means that updates the classification information based on the determination result, the first evaluation result, and the second evaluation result stored by the storage means.

13. The biometric information computing system according to claim 1, comprising:
a server that stores the database and generates the first evaluation result by the generating means; and
a biometric information computing device that receives the first evaluation result from the server and displays the first evaluation result.

14. A server that stores the first evaluation result according to claim 1.

15. A data structure used by a computer that includes a display unit, a control unit, and a storing unit, the data structure being stored in the storing unit, the data structure comprising
the first evaluation result and the second evaluation result generated by the biometric information computing system according to claim 2, wherein
the first evaluation result and the second evaluation result are used when the control unit generates a comprehensive evaluation result comprehensively evaluating a feature of the user.
